(19) **Europäisches Patentamt**
**European Patent Office**
**Office européen des brevets**

(11) **EP 1 664 077 B1**

(12) **EUROPEAN PATENT SPECIFICATION**

(45) Date of publication and mention
of the grant of the patent:
**13.04.2016 Bulletin 2016/15**

(21) Application number: **04783186.2**

(22) Date of filing: **07.09.2004**

(51) Int Cl.:
***C12Q 1/68*** (2006.01)

(86) International application number:
**PCT/US2004/028857**

(87) International publication number:
**WO 2005/023091 (17.03.2005 Gazette 2005/11)**

(54) **METHOD FOR NON-INVASIVE PRENATAL DIAGNOSIS**

VERFAHREN ZUR NICHTINVASIVEN PRÄNATALEN DIAGNOSE

PROCEDE DE DIAGNOSTIC PRENATAL NON EFFRACTIF

(84) Designated Contracting States:
**AT BE BG CH CY CZ DE DK EE ES FI FR GB GR
HU IE IT LI LU MC NL PL PT RO SE SI SK TR**

(30) Priority: **05.09.2003 US 500526 P**

(43) Date of publication of application:
**07.06.2006 Bulletin 2006/23**

(60) Divisional application:
**10189690.0 / 2 354 253**

(73) Proprietors:
• **Trustees of Boston University
Boston, MA 02215 (US)**
• **THE CHINESE UNIVERSITY OF HONG KONG
New Territories
Hong Kong (CN)**

(72) Inventors:
• **CANTOR, Charles, R.
Del Mar, CA 92014 (US)**
• **DING, Chunming
Waltham, MA 02453 (US)**
• **LO, Yuk Ming Dennis
Kowloon,
Hong Kong (CN)**
• **CHIU, Rossa, Wai-Kwun
Tai Po, N.T.,
Hong Kong (CN)**

(74) Representative: **O'Connell, David Christopher
Haseltine Lake LLP
Redcliff Quay
120 Redcliff Street
Bristol BS1 6HU (GB)**

(56) References cited:
**WO-A-01/75163**

• **TANG K ET AL: "Chip-based genotyping by mass
spectrometry." PROCEEDINGS OF THE
NATIONAL ACADEMY OF SCIENCES OF THE
UNITED STATES OF AMERICA 31 AUG 1999, vol.
96, no. 18, 31 August 1999 (1999-08-31), pages
10016-10020, XP002447905 ISSN: 0027-8424**
• **CHIU ET AL: 'Prenatal exclusion of beta
thalassaemia major by examination of maternal
plasma' THE LANCET September 2002, pages 998
- 1000, XP004794194**
• **AMICUCCI ET AL: 'Prenatal Diagnosis of
myoptonic dystrophy using fetal DNA obtained
from Maternal plasma' CLINICAL CHEMISTRY
vol. 46, no. 2, February 2000, pages 301 - 302,
XP008090463**
• **SAITO ET AL: 'Prenatal DNA diagnosis of
single-gene disorder from maternal plasma' THE
LANCET vol. 356, September 2000, page 1170,
XP004815122**
• **GRANT WU ET AL: "Rapid, accurate genotyping
of beta-thalassaemia mutations using a novel
multiplex primer extension/denaturing
high-performance liquid chromatography
assay.", BRITISH JOURNAL OF HAEMATOLOGY,
vol. 122, no. 2, 1 July 2003 (2003-07-01), pages
311-316, XP55031458, ISSN: 0007-1048**

## Description

## BACKGROUND

The analysis of circulating nucleic acids has revealed applications in the noninvasive diagnosis, monitoring, and prognostication of many clinical conditions.

For example, in non-invasive methods for prenatal monitoring, fetal DNA has been found to circulate in maternal plasma (Lo, Y. M. D. et al. Lancet 350,485-487 (1997)), and development of such non-invasive prenatal diagnosis has therefore been suggested based on the analysis of a maternal blood sample. Although the non-invasive nature of such approaches represents a major advantage over conventional methods. However, the technical challenge posed by the analysis of fetal DNA. Thus, in maternal plasma lies in the need to be able to discriminate the fetal DNA from the co-existing background maternal DNA, and the diagnostic reliability of circulating DNA analysis depends on the fractional concentration of the targeted sequence, the analytical sensitivity, and the specificity of the method.

Fetal DNA represents a minor fraction of the total DNA in maternal plasma, contributing approximately 3% to 6% of the total maternal plasma DNA in early and late pregnancy, respectively (Lo, Y. M. D. et al. Am J Hum Genet 62, 768-775 (1998)).

Most diagnostic applications reported to date have focused on detecting of paternally-inherited fetal traits or mutations, as these are more readily distinguishable from

the background maternal DNA. Reported applications include the prenatal diagnosis of sex-linked diseases (Costa, J.M., Benachi, A. & Gautier, E. N Engl J Med 346, 1502 (2002)), fetal RhD status (Lo, Y.M.D. et al. N Engl JMed 339, 1734-1738 (1998)) and certain paternally-transmitted autosomal dominant conditions, including achondroplasia and myotonic dystrophy (Chiu, R.W.K. & Lo, Y.M.D. Expert Rev Mol Diagn 2, 32-40 (2002)).

Fetal SRY and RHD DNA detection from maternal plasma has reached close to 100% accuracy, as confirmed by many large scale evaluations (Sekizawa, A., Kondo, T., Iwasaki, M., Watanabe, A., Jimbo, M., Saito, H. & Okai, T. (2001) Clin. Chem. 47, 1856-1858; Finning, K. M., Martin, P. G., Soothill, P. W. & Avent, N. D. (2002) Transfusion 42, 1079-1085; Costa, J. M., Benachi, A., Gautier, E., Jouannic, J. M., Ernault, P. & Dumez, Y. (2001) Prenatal Diagn. 21, 1070-1074; Rijnders, R. J., Christiaens, G. C., Bossers, B., van der Smagt, J. J., van der Schoot, C. E. & de Haas, M. (2004) Obstet. Gynecol. 103, 157-164). However, its general applicability is limited. The high level of diagnostic accuracy in these conditions is attained by the analytical sensitivity contributed by the use of real-time quantitative PCR (Lo Y et al. Am. J. Hum. Genet. 62:768-775, 1998; Heid et al., Genome Res. 6:986-994, 1996), and the analytical specificity conferred choosing fetal DNA targets that are absolutely fetal-specific. The RHD sequence does not exist in the genome of a rhesus D negative mother, and SRY, which is used to detect the presence of a Y chromosome, does not exist in a genome of a normal woman. Consequently, the maternal plasma SRY and RHD analyses are relatively free from interference by the background maternal DNA. This differs from a number of other conditions.

Many fetal genetic diseases are caused by mutations that result in more subtle genetic differences between the maternal and fetal DNA sequences in maternal plasma. While such fetal diseases may theoretically be diagnosed non-invasively by means of the detection or exclusion of the paternally inherited mutant allele in maternal plasma, the development of robust assays for the discrimination of less dramatic differences between fetal and maternal DNA in maternal plasma has been technically challenging (Nasis, O., Thompson, S., Hong, T., Sherwood, M., Radcliffe, S., Jackson, L. & Otevrel, T. (2004) Clin. Chem. 50, 694-701). Therefore, despite many potential applications reported for fetal mutation detection in maternal plasma, such as achondroplasia, Huntington's disease, cystic fibrosis, and hemoglobin E (Nasis, O., Thompson, S., Hong, T., Sherwood, M., Radcliffe, S., Jackson, L. & Otevrel, T. (2004) Clin. Chem. 50, 694-701; Saito, H., Sekizawa, A., Morimoto, T., Suzuki, M. & Yanaihara, T. (2000) Lancet 356, 1170; Gonzalez-Gonzalez, M. C., Trujillo, M. J., Rodriguez de Alba, M. & Ramos, C. (2003) Neurology 60, 1214-1215; Gonzalez-Gonzalez, M. C., Garcia-Hoyos, M., Trujillo, M. J., Rodriguez de Alba, M., Lorda-Sanchez, I., Diaz-Recasens, J., Gallardo, E., Ayuso, C. & Ramos, C. (2002) Prenatal Diagn. 22, 946-948; Fucharoen, G., Tungwiwat, W., Ratanasiri, T., Sanchaisuriya, K. & Fucharoen, S. (2003) Prenatal Diagn. 23, 393-396), most published data only involve case reports of isolated patients. Large-scale evaluation of analytical protocols for circulating fetal DNA discrimination has been limited. Reliable discrimination between the fetal and maternal DNA sequences would depend heavily on the analytical specificity of the assay system. The degree of analytical specificity required for accurate analysis is inversely related to the degree of genetic difference between the alleles of interest and the background DNA (Lo, Y. M. D. (1994) J. Pathol. 174, 1-6). Thus a need exists for methods that can reliably analyze such subtle genetic differences.

The prenatal assessment of autosomal recessive diseases based on fetal DNA analysis in maternal plasma presents another challenge. The manifestation of an autosomal recessive disease results from the inheritance of a mutant allele from each parent. Thus, an autosomal recessive condition could either be confirmed prenatally through the demonstration of the inheritance of two mutant alleles, or could be excluded by the demonstration of the inheritance of at least one non-mutant allele. The current strategies look at exclusion. For example, one such strategy is based on the haplotype assessment of polymorphisms associated with the paternally-inherited non-mutant allele (Chiu, R.W.K. et al. Clin Chem 48, 778-780 (2002)).

β-thalassemia is an autosomal recessive condition resulting from the reduced or absent synthesis of the β-globin chains of hemoglobin. It is highly prevalent in the Mediterranean, the Middle East, the Indian subcontinent and Southeast Asia (Weatherall, D.J. & Clegg, J.B. Bull World Health Organ 79, 704-712 (2001)). More that 200 β-thalassemia mutations have been described, many of which are point mutations (Weatherall, D. J. (1997) BMJ314, 1675-1678). β-thalassemia major is an otherwise lethal condition where survival is dependent on life-long blood transfusions and iron chelation therapy. Curative therapies are not readily available and therefore, much focus has been devoted to disease prevention through prenatal diagnosis.

The alpha and beta loci determine the structure of the 2 types of polypeptide chains in adult hemoglobin, Hb A. Mutant beta globin that sickles causes sickle cell anemia (http://www.ncbi.nlm.nih.gov/entrez/dispomim). Absence of the beta chain causes beta-zero-thalassemia. Reduced amounts of detectable beta globin causes beta-plus-thalassemia, which is one of the most common single gene disorders in the world.

For clinical purposes, beta-thalassemia is divided into thalassemia major (transfusion dependent), thalassemia intermedia (of intermediate severity), and thalassemia minor (asymptomatic). Patients with thalassemia major present in the first year of life have severe anemia; they are unable to maintain a hemoglobin level above 5 gm/dl. Clinical details of this disorder have been detailed extensively in numerous monographs and are summarized by Weatherall, et al. (The hemoglobinopathies. In: Scriver, C.; Beaudet, A. L.; Sly, W. S.; Valle, D. (eds.) : The Metabolic and Molecular Bases of Inherited Disease. (7th ed.) New York: McGraw-Hill 1995. Pp. 3417-3484). The prognosis for individuals with beta-thalassemia is very poor. For example, in 2000 it was reported that about 50% of U.K. patients with beta-thalassemia major die before the age of 35 years, mainly because conventional iron-chelation therapy is too burdensome for full adherence (Model et al. Survival in beta-thalassaemia major in the UK: data from the UK Thalassaemia Register. Lancet 355: 2051-2052, 2000).

The molecular pathology of disorders resulting from mutations in the nonalpha-globin gene region is the best known, this elucidation having started with sickle cell anemia in the late 1940s. Steinberg and Adams reviewed the molecular defects identified in thalassemias: (1) gene deletion, e.g., of the terminal portion of the beta gene (2) chain termination (nonsense) mutations; (3) point mutation in an intervening sequence; (4) point mutation at an intervening sequence splice junction; (5) frameshift deletion; (6) fusion genes, e.g., the hemoglobins Lepore; and (7) single amino acid mutation leading to very unstable globin, e.g., Hb Vicksburg (beta 75 leuto-0) Steinberg, M. H.; Adams, J. G., III : Thalassemia: recent insights into molecular mechanisms. Am. J. Hemat. 12: 81-92, 1982.

Because of the frequency of the mutations in the populations and the devastating clinical, symptoms including the markedly reduced life span, prenatal diagnosis is important. For example, it can provide a means for disease prevention. However, the conventional methods of prenatal diagnosis such as, amniocentesis, chorionic villus sampling and cordocentesis, are all associated with a small but finite risk of fetal loss. Therefore, it would be important to develop a non-invasive method for prenatal diagnosis of thalassemias. Attempts have been made in the past to develop other means of non-invasive prenatal diagnosis of β-thalassemia, including the analysis of fetal cells in maternal blood (Cheung, M.C., Goldberg, J.D. & Kan, Y.W. Nat Genet 14, 264-268 (1996)). However, these methods are labor-intensive and time-consuming. Consequently, the need exists to develop tools that accurately permit highly specific and sensitive detection of nucleic acids in biological samples, particularly parentally inherited alleles.

## SUMMARY

Accordingly, the present invention is directed to methods of detecting nucleic acids in a biological sample.

We show the feasibility of the use of mass spectrometric analysis for the discrimination of fetal point mutations in maternal plasma and developed an approach for the reliable exclusion of mutations in maternal plasma. We further show the feasibility of the' approach for the minimally invasive prenatal diagnosis in a situation where a mother and father share an identical disease causing mutation, a concurrence previously perceived as a challenge for maternal plasma-based prenatal diagnosis for autosomal recessive diseases.

In one embodiment, the invention is directed to a method for the detection of paternally-inherited fetal-specific β-thalassemia mutations in maternal plasma based on methods for looking at nucleic acid segments using methods such as the primer-extension of polymerase chain reaction (PCR) products, at about single molecule dilution. This is preferably followed by mass spectrometric detection. The technique allows the non-invasive prenatal exclusion of β-thalassemia with high throughput capacity and is applicable to any disease caused by mutations in a single gene including, but not limited recessive single gene diseases such as thalassemias, such as beta thalassemias, cystic fibrosis, and congenital adrenal hyperplasia.

The invention is based upon a discovery that a highly sensitive and specific mutation-specific analysis of the paternally-inherited mutation in maternal plasma can be used to exclude the fetal inheritance of the paternal mutation based on its negative detection. For example, using a real-time quantitative allele-specific polymerase chain reaction (PCR) approach to exclude the inheritance of the β-thalassemia mutation codons (CD) 41/42 (- CTTT), involving the deletion of four nucleotides (CTTT) between codons 41 and 42 of the, *β-globin* gene, *HBB* (Chiu, R. W. K. et al. Lancet 360, 998-1000 (2002)), shows that the negative exclusion proposed herein can readily be used.

To achieve single nucleotide discrimination at low fractional concentrations, an analytical system that combines the use of an approach with better allele-specificity and high detection sensitivity is required. One example is the use of primer extension analysis in a system such as the MassARRAY system (SEQUENOM), that allows a high throughput approach for the detection and exclusion of paternally-inherited fetal mutations in maternal plasma with the capability of single base discrimination. The MassARRAY system is based on matrix-assisted laser desorption ionization/time-of-flight (MALDI-TOF) mass spectrometric (MS) analysis of primer-extension products (Tang, K. et al. Proc Natl Acad Sci USA 96, 10016-10020 (1999)).

In one embodiment, the invention is directed to a method of detecting a genetic disorder in a fetus from a blood, serum or plasma sample of a pregnant woman, the method comprising: a) analyzing both isolated maternal and paternal DNA for a disease-causing mutation for the single gene disorder; b) if both maternal and paternal nucleic acid, e.g., DNA carry a disease causing mutation for the same disease then isolating the nucleic acid, e.g., DNA from plasma, blood, or serum of the pregnant mother; c) determining a fetal genotype from the isolated maternal plasma DNA using primers corresponding to the paternally identified mutation and performing a mutation-specific primer-extension assay in at least 15, preferably several replicates, for example about 20, 25-100 replicates and even up to about 1000 replicates. Most preferably about 15-25 replicates are used, wherein a detection of the paternal mutation in any of the replicate sample is indicative of the presence of the single-gene disorder in the fetus.

In one preferred embodiment, the single gene disease is an autosomal recessive disease. In the most preferred embodiment, the autosomal recessive disease is selected from beta thalassemias, cystic fibrosis and congenital adrenal hyperplasia. In the most preferred embodiment, the disease is beta thalassemia caused by mutations selected from the group consisting of CD 41/42-CTTT; IVS2 654 (C-> T); nucleotide-28 (A-> G); and CD 17 (A-> T).

In the preferred embodiment the number of replicates is at least 15, preferably at least about 15-25, or 25-100, up to at least about 1000 replicates. Most preferably the number of replicates is about 15-25.

In one embodiment, the primer-extension analysis is performed using the MassARRAY system (SEQUENOM).

The invention enables a method of detecting a genetic disease in a fetus using maternally isolated DNA from plasma, serum, or blood, the method comprising: a) selecting one or more single nucleotide polymorphisms (SNP) which are not disease-causing polymorphisms and which are associated either with a paternal disease-causing allele or with a paternal healthy allele and which SNP differs between the maternal and the paternal genotype; b) determining the fetal genotype from a sample DNA isolated from the plasma, serum or whole blood of the pregnant mother, wherein the determination is performed using primers corresponding to both the selected SNP and the disease-causing mutation and performing an SNP and disease mutation-specific primer-extension assay in several replicates using said primers; c) wherein detection of the SNP associated with the paternal allele in any of the replicate samples is indicative of the presence of the paternal allele inherited by the fetus and the detection of the paternal disease-causing mutation in any of the replicate samples indicates detection of the genetic disease inherited by the fetus, wherein detection of the SNP associated with the healthy paternal allele in any of the replicate samples is indicative of the presence of the healthy allele inherited by the fetus and excludes the inheritance of the genetic disease by the fetus.

**BRIEF DESCRIPTION OF DRAWINGS**

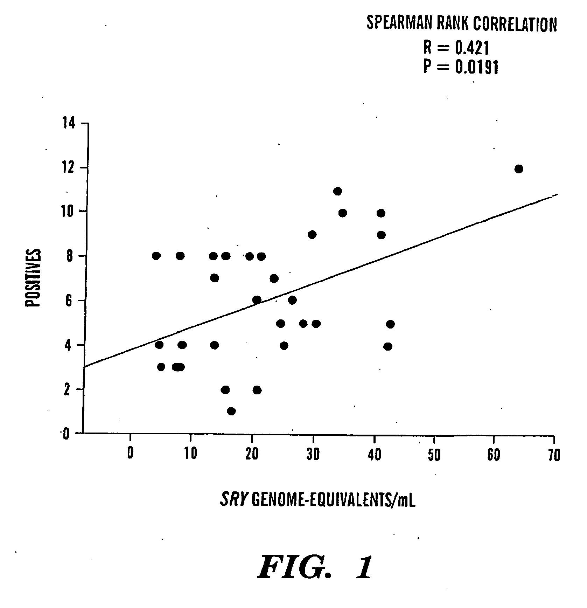

    **Figure 1** demonstrates the relationship between the number of positives detected in the 15-replicate PCR experiments for fetal gender determination (y-axis) and the fetal DNA concentration measured by real-time quantitative PCR targeting the Y-chromosome gene, SRY (x-axis). The theoretical basis of using the 15-replicate format is based on the Poisson distribution of fetal DNA molecules at single molecule concentration. The equation for the Poisson distri-

bution is: $P(n) = \dfrac{m^n e^{-m}}{n!}$, where, n= number of fetal DNA molecules per PCR, P(n)=probability of n fetal DNA molecules in a particular PCR; m= mean number of fetal DNA molecules in a particular plasma DNA sample.

Using our standard plasma DNA extraction protocol (see text), each PCR using 1 $\mu$L of maternal plasma DNA contains 0.4 genome-equivalent of the paternally-inherited fetal DNA. Thus, the probability that a particular PCR will be negative due to having no fetal DNA molecule is:

$$P(0) = \frac{0.4^0 e^{-0.4}}{0!} = 0.670$$

To reduce the false-negative rate, the probability that all replicates are negative for a 15-replicate PCR experiment is: $0.670^{15} = 0.0025$.

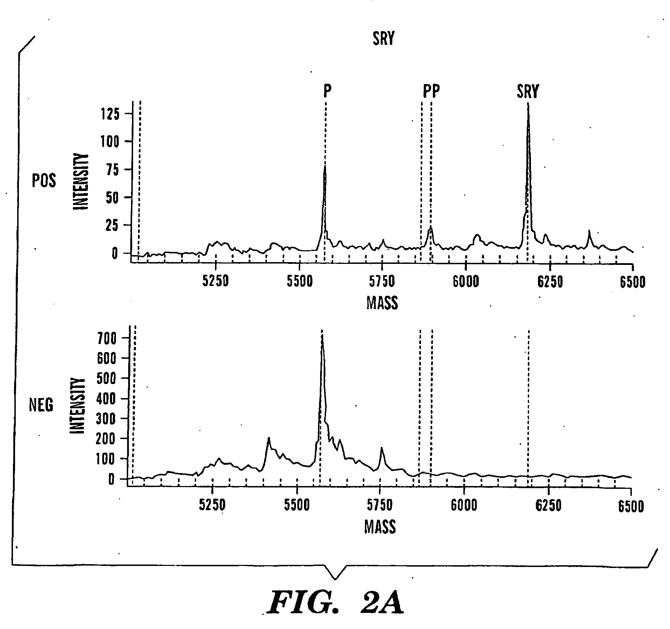

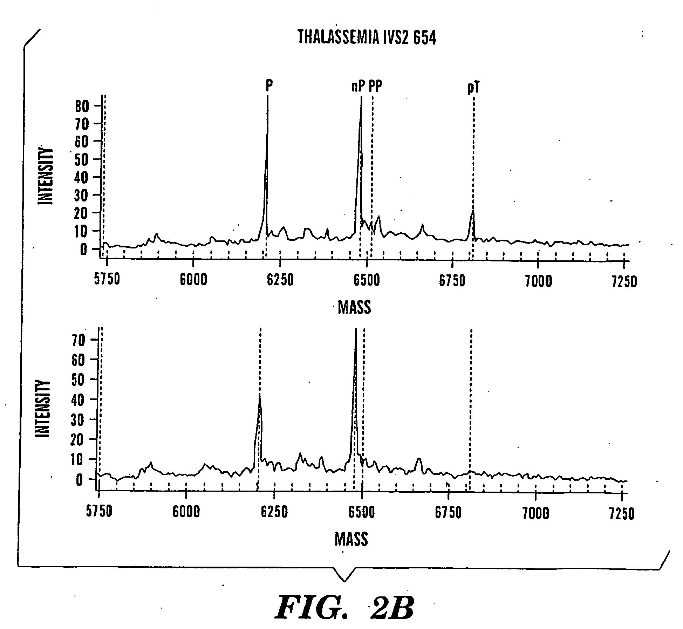

    **Figures 2A and 2B** show the Mass Spectrometric analyses of the SRY DNA and the paternally inherited thalassemia IVS2 654 mutation. Mass spectra for the other three thalassemia mutations are similar. For all mass spectra, Mass (x-axis) represents the molecular weight of the marked peaks. The molecular weights of all relevant peaks are calculated before the analysis and the Mass values measured by mass spectrometry are generally only 0-5 Dalton off. Intensity (y-axis) is of an arbitrary unit. P and PP represent unextended primer and pausing product (i.e., premature termination of the base extension reaction), respectively. For SRY DNA analysis, the SRY peak is present (thus a positive result, marked as POS at the left side of the figure) in some of the 15 replicates (see Fig. 1). None of the 15 replicates has a SRY peak (thus a negative result, marked as NEG in the figure), if a women was pregnant with a female fetus. For thalassemia IVS2 654 mutation analysis, the pT peak is from the paternally inherited thalassemia IVS2 654 mutation and is present in some of the 15 replicates for fetuses carrying a paternally inherited thalassemia IVS2 654 mutation (see Table 1). The nP peak is from all other $\beta$-globin alleles except the paternally inherited thalassemia IVS2 654 allele.

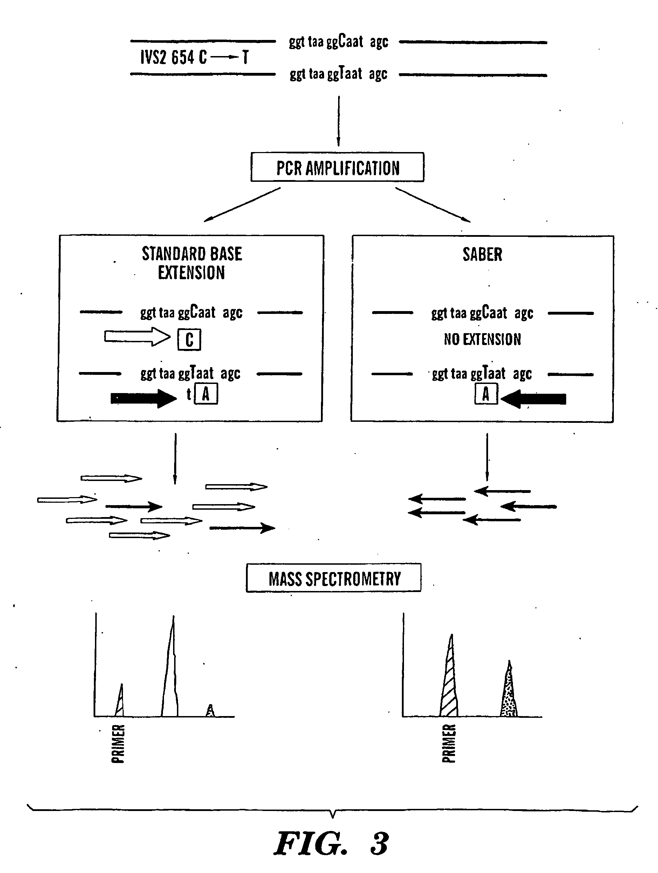

**Figure 3** shows a schematic illustration of the single allele base extension reaction (SABER) and standard MassARRAY assays. Maternal plasma detection of the paternally inherited fetal-specific $\beta$-thalassemia mutation, IVS2 654 C $\rightarrow$T, is presented as an illustrative example. Maternal plasma is first amplified by PCR. The PCR products are subjected to base extension by the standard and SABER protocols. The standard protocol involves the base extension of both the mutant fetal allele (T allele) and the background allele (C allele), whereas the SABER method only extends the fetal-specific mutant allele. The base extension reactions are terminated by dideoxynucleotides, indicated in boxes. The extension products of the standard protocol include a predominance of the nonmutant allele (open arrows) with a small fraction of the fetal-specific mutant allele (filled arrows). The low abundance of the fetal allele (filled peak) is overshadowed by the nonmutant allele (open peak) on the mass spectrum. Because SABER only involves the extension of the mutant allele, the latter's presence (filled peak) can be robustly identified from the mass spectrum. The striped peaks represent the unextended primer.

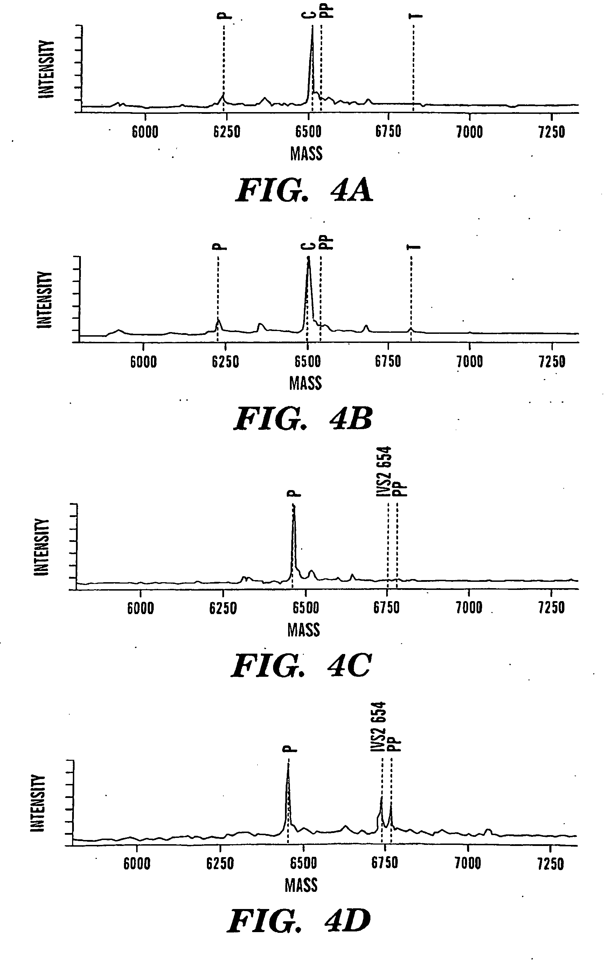

**Figures 4A-4D** show the MS analyses of the paternally inherited $\beta$-thalassemia IVS2 654 mutation in maternal plasma. For all mass spectra, mass (x axis) represents the molecular weight of the marked peaks. The expected molecular weights of all relevant peaks were calculated before the analysis. Intensity ($y$ axis) is in arbitrary units. P and PP, unextended primer and pausing product (i.e., premature termination of the base extension reaction or incorporation of an undigested dGTP from shrimp alkaline phosphatase treatment for the wild-type DNA template), respectively. Figs 4A and 4B illustrate the mass spectra obtained by the standard MassARRAY protocol for a fetus negative and positive for the mutation, respectively. T, expected mass of the mutant allele; C, position of the alleles without the IVS2 654 mutation. Figs 4C and 4D illustrate the mass spectra obtained by the SABER MassARRAY protocol for a fetus negative and positive for the mutation, respectively. IVS2 654, expected mass of the mutant allele.

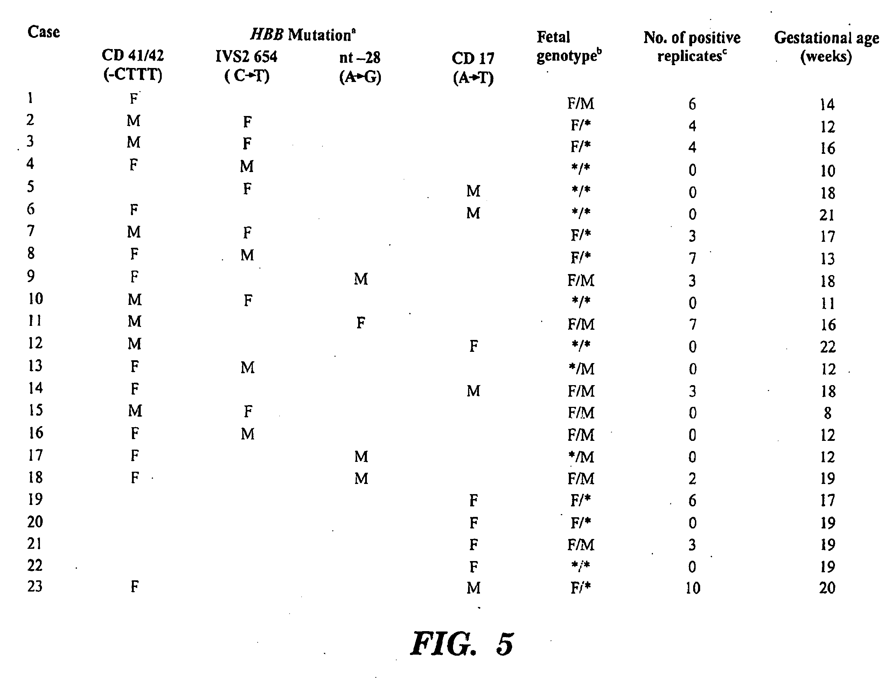

**Figure 5** shows Table 1, showing prenatal exclusion of $\beta$-thalassemia major by maternal plasma analysis. All of the parents are carriers for $\beta$-thalassemia and have one *HBB* mutation. The [a]mutations of the father and mother are marked by "F' and "M", respectively. The maternal mutation is not indicated for cases where the maternal mutation is not one of the four *HBB* mutations studied. The [b]fetal genotype is indicated by the inheritance of the paternal mutation "F', the maternal mutation "M", or the normal allele "*". Results of the MassARRAY maternal plasma analysis is indicated by the [c]number of replicates among the 15 repeats where the paternally-inherited fetal allele was positively detected. The fetus is deemed to have inherited the paternal mutation if any of the 15 repeats showed a positive result.

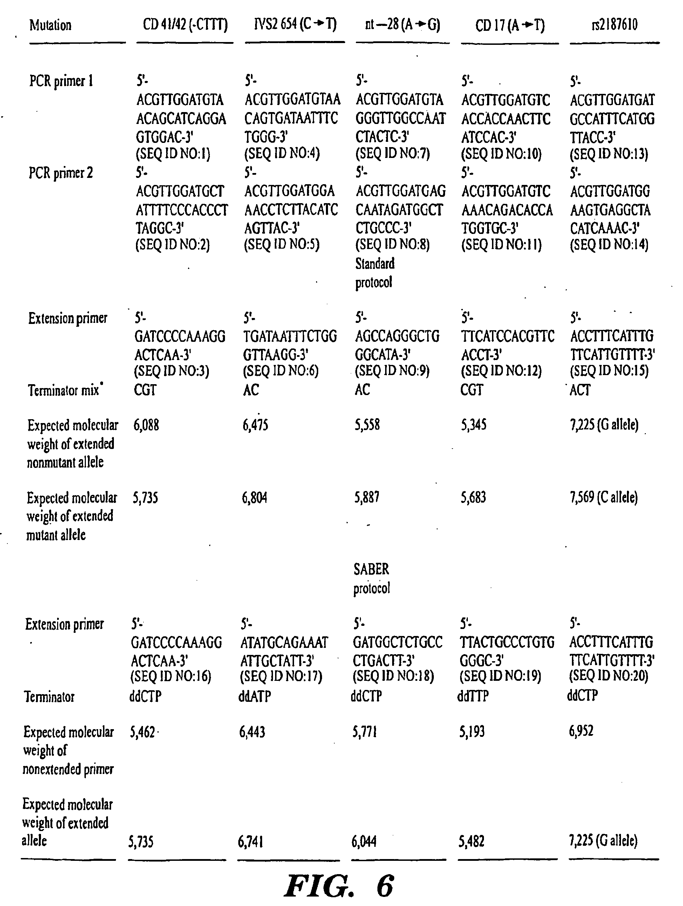

**Figure 6** shows Table 2 including PCR and extension primer sequences. *CCT mix is ddCTP/ddGTP/ddTTP/dATP in which dd indicates the 2',3'-dideoxynucleoside. Similarly AC mis is ddATP/ddCTP/dGTP/dTTP.

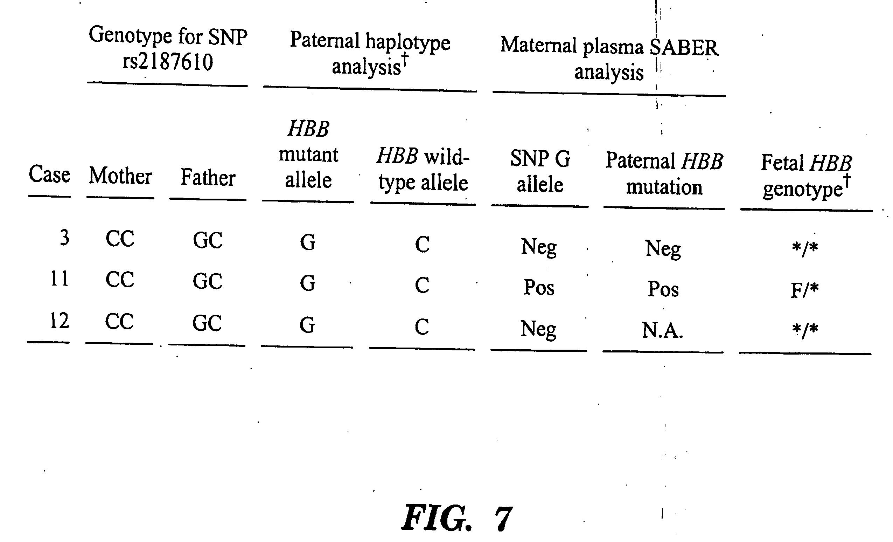

**Figure 7** shows Table 3 including data from detection of paternally inherited *HBB* mutations in maternal plasma. All the patients are carriers of $\beta$-thalassemia and have one *HBB* mutation. The maternal mutation is not indicated for cases where the maternal mutation is not one of the four *HBB* mutations studies. F and M, mutations of the father and mother, respectively; -, no mutation; neg, negative; pos, positive; N.A. not applicable. †the fetal genotype determined by conventional methods is indicated by the inheritance of the paternal mutation F, the maternal mutation M, or the normal allele, *.

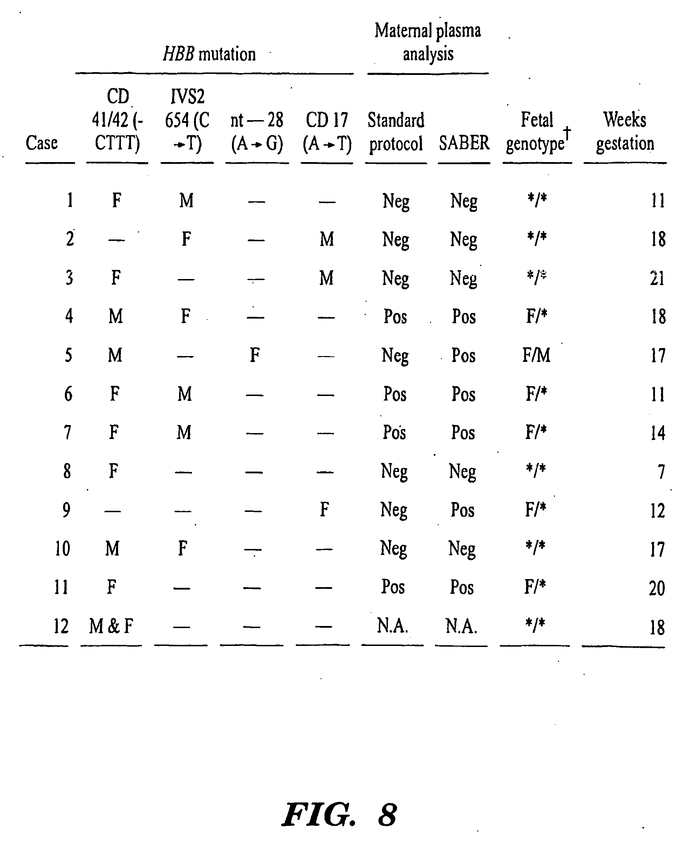

**Figure 8** shows Table 4 including data from haplotype analysis of paternally inherited alleles in maternal plasma. Neg, negative; Pos, positive; N.A., not applicable. †G and C denote the rs2187610 allele linked to the mutant or wild-type paternal *HBB* alleles, respectively. ‡ The fetal genotype determined by conventional methods is indicated by the inheritance of the paternal mutation F, the maternal mutation M, or the normal allele, *.

## DETAILED DESCRIPTION

The present invention is directed to methods of detecting nucleic acids in a biological sample. The method may be based on a novel combination of a base extension reaction, which provides excellent analytical specificity, and a mass spectrometric analysis, which provides excellent specificity. The method can be used, for example, for diagnostic, prognostic and treatment purposes. The method allows accurate detection of nucleic acids that are present in very small amounts in a biological sample. The method of the present invention is used to detect fetal nucleic acid in a maternal blood sample.

The methods provided are minimally invasive, requiring generally, a small amount of a biological sample, for example, a blood, plasma, serum, urine, bucchal or nasal swap, saliva, skin scratch, hair or stool sample from an individual.

In the case of determining a fetal genotype or quantitating the fetal nucleic acids or alleles using the methods of the present invention, the sample is a maternal tissue sample available without posing a risk to the fetus. Such biological material is maternal blood, plasma or serum.

In the present study, we evaluated, and show the feasibility of, the use of mass spectrometry (MS) for the discrimination of fetal point mutations in maternal plasma and developed an approach for the reliable exclusion of mutations in maternal plasma. We further evaluated, and show the feasibility of, the approach for the noninvasive prenatal diagnosis of a mother and father sharing an identical disease causing mutation, an occurrence previously perceived as a challenge for maternal plasma-based prenatal diagnosis for autosomal recessive diseases.

The methods of the present invention are automatable. For example, use of mass spectrometry, such as MassARRAY system (Sequenom Inc, CA), in combination with the present invention allows analysis of fetal DNA with the capacity of over 2000 samples per day in triplicate samples thus making the method a practical system for routine use.

In one preferred embodiment, the invention provides an accurate method for determining differences between fetal and maternal nucleic acids in a maternal blood sample allowing for a minimally invasive and reliable method for prenatal diagnosis. The method is based on a combination of a base extension reaction and a mass spectrometric analysis. Thus, prenatal diagnosis can be performed without the potential complications to the fetus and the mother that are associated with traditional methods for prenatal diagnosis including amniotic fluid and/or chorionic villus sampling.

Due to the specificity of the base extension reaction, allelic differences can be accurately amplified for analysis including changed varying from single nucleotide variations to small and large deletions, insertions, invertions and other types of nucleic acid changes that occur in even a small percentage of the pool of nucleic acids present in a sample.

The base extension reaction which may be used in the present invention can be performed using any standard base extension method. In general, a nucleic acid primer is designed to anneal to the target nucleic acid next to or close to a site that differs between the different alleles in the locus. In the standard base extension methods, all the alleles present in the biological sample are amplified, when the base extension is performed using a polymerase and a mixture of deoxy-and dideoxycynucleosides corresponding to all relevant alleles. Thus, for example, if the allelic variation is A/C, and the primer is designed to anneal immediately before the variation site, a mixture of ddATP/ddCTP/dTTP/dGTP will allow amplification of both of the alleles in the sample, if both alleles are present. Table 2 in Figure 6 shows exemplary mixtures for the standard base extension reactions for detecting several different nucleic acid variations in the *HBB* locus.

After the base extension reaction, the extension products including nucleic acids with A and C in their 3' ends, can be separated based on their different masses. Alternatively, if the ddNTPs are labeled with different labels, such as radioactive or fluorescent labels, the alleles can be differentiated based on the label. In a preferred embodiment, the base extension products are separated using mass spectrometric analysis wherein the peaks representing different masses of the extension products, represent the different alleles.

In one embodiment, the base extension is performed using single allele base extension reaction (SABER, Fig. 3). In SABER, one allele of interest per locus is amplified in one reaction by adding only one dideoxynucleotide corresponding to the allele that one wishes to detect in the sample. One or more reactions can be performed to determine the presence of a variety of alleles in the same locus. Alternatively, several loci with one selected allele of interest can be extended in one reaction.

The specificity provided by primer extension reaction, particularly SABER, allows accurate detection of nucleic acids with even a single base pair difference in a sample, wherein the nucleic acid with the single base pair difference is present in very small amounts. For example, fetal nucleic acids have been generally found to represent only about 3-6% of the nucleic acids circulating in the maternal blood (Lo et al, Am J Hum Genet 62, 768-775, 1998). We have now shown that the methods provided by the present invention can be used to detect polymorphisms present in the fetal nucleic acids from a sample taken from the pregnant mother.

Therefore, in one embodiment, the invention provides a method for detecting fetal nucleic acids in maternal blood. The method comprises obtaining a nucleic acid sample from the pregnant mother and analyzing the sample using base extension and subsequent mass spectrometric analysis to detect one or more loci of the fetal nucleic acid in the sample.

In one embodiment, the invention provides a method for detecting a paternally-inherited mutations in the fetus from the maternal blood. The method comprises analyzing the paternal nucleic acid sample and determining the presence of one or more paternal nucleic acid polymorphisms. The maternal blood/plasma sample is then analyzed for the presence or absence of the paternally inherited allele using base extension, preferably SABER, wherein only ddNTP corresponding to the paternal mutation(s) is used in the base extension reaction. The base extension products are then detected using any detection methods, that can differentiate between the base extended nucleic acid products. Preferably, the detection is performed using matrix assisted laser desorption ionization/time-of-flight mass spectrometric analysis, for example, as described in Example 2. The presence and/or absence of the paternal alleles in the maternal blood/plasma sample is exemplified in Figure 4, wherein the presence of the peak representing the paternally inherited IVS2 654 (C→T) can be seen in Fig. 4D and absence of the same allele in Fig. 4C.

The method can be used to reliably detect paternally inherited disease causing mutations including any dominant or recessive diseases such as achondroplasia, Huntington's disease, cystic fibrosis, hemoglobin E and the different hemoglobinopathies, such as β-thalassemia. Based on this disclosure, a skilled artisan will be able to design a detection method for prenatal diagnosis for any disease wherein the disease causing mutation or a genetic polymorphism(s) associated with or linked to the disease is/are known.

We have illustrated the reliability of the SABER assays for single-nucleotide discrimination between circulating fetal and maternal DNA by the maternal plasma detection of fetal β-thalassemia point mutations and SNPs. The ability to robustly analyze fetal-specific SNPs in maternal plasma is a useful adjunct procedure for maternal-plasma fetal DNA analysis as a safeguard against the possibility of false-negative detection due to fetal DNA degradation, DNA extraction failures, or PCR allele dropout. With the availability of a reliable MS method for fetal single nucleotide polymorphism (SNP) detection in maternal plasma, the number of potential gender-independent internal control targets for circulating fetal DNA detection has increased substantially.

Circulating fetal SNPs can also be analyzed according to the method of the present invention,. This permits fetal haplotype analysis from maternal plasma. Noninvasive fetal haplotyping can be achieved by means of analyzing polymorphisms linked to a mutated locus. Haplotype analysis between the *HBB* locus and a linked polymorphism premits the noninvasive prenatal exclusion of β-thalassemia major, despite the presence of the same *HBB* mutation in both parents. See, for example, case 12 of Example 2 below. This procedure overcomes the previously perceived deficiency in maternal plasma-based prenatal diagnosis of autosomal recessive diseases that limited its applicability to couples sharing different mutations.

Therefore, the haplotype approach also can be applied to maternal plasma detection of a fetal SNP allele linked to the paternal nonmutant allele. The positive detection of such an allele results in the noninvasive positive prenatal exclusion of a disease, such as β-thalassemia major and other recessive diseases wherein mother and father share the same mutation but carry different SNPs. Example 2 shows the results of non-invasive fetal haplotyping in a β-thalassemia case using an informative polymorphism at locus rs2187610.

Thus, the invention provides a method for determining a fetal haplotype to determine the presence of paternally inherited allele at any given locus in the fetal genome from the maternal plasma/blood. The method comprises the steps of determining one or more polymorphisms that differ between maternal and paternal genomes, i.e., SNPs that are informative. The SNPs should be linked to the locus wherein determination of any given allele is desired. The determination of informative SNPs can be performed using any genotyping methods routinely available for a skilled artisan. Any haplotyping methods can be used. In one preferred embodiment, direct molecular haplotyping method is used as explained below (see, Ding and Cantor, Proc Natl Acad Sci USA, 100: 7449-7453, 2003). The fetal nucleic acid is analyzed from the maternal blood/plasma using the method of the present invention comprising amplification of the nucleic acids, for example using PCR, performing a base extension reaction, preferably SABER, followed by detection of the base extension products, preferably using MS based techniques.

Haplotyping of the fetal nucleic acid allows accurate prenatal diagnosis of a disease wherein both parents may be carriers of the same mutation, but carry that mutation in only one allele. Therefore, for example, if the disease is recessive, determination of one healthy allele in the fetal nucleic acid shows that the fetus, if born, will not be affected with the disease.

In one embodiment, the method of the invention is applied to quantification of fetal nucleic acids from the maternal biological sample. Quantitative aberrations in circulating fetal DNA concentrations have been demonstrated for fetal chromosomal aneuploidies, preeclampsia, preterm labor, and many other pregnancy associated complications. Therefore, the present method permits for determining the risk of aneuploidies, preeclampsia, preterm labor, and other pregnancy associated complications. The methods use the principle of analyzing the presence of fetal specific paternally inherited allele determined using nucleic acid amplification, base extension and analysis of the base extension products as described elsewhere in the specification. Quantification is consequently performed either by comparing the ratio of the maternal allele and the fetal specific allele or by including an external standard in known amounts to determine the amount or relative amount of the fetal nucleic acid in the sample. Because the method of the invention allows minimally invasive sample collection, the comparison can be performed either at one desired time during pregnancy or several times during the parts or entire pregnancy to allow a follow-up of the fetal condition throughout the pregnancy.

In one embodiment, the invention provides a method for the detection of paternally-inherited fetal-specific β-thalassemia mutations in maternal plasma based on a method of analyzing the paternal nucleic acid at single molecule dilution. Preferably, the analysis is performed using a primer-extension of polymerase chain reaction (PCR) and detecting the primer extension products using mass spectrometry. Alternatively, the detection can be performed using, for example, electrophoretic methods including capillary electrophoresis, using denaturing high performance liquid chromatography (D-HPLC), using an Invader® Assay (Third Wave Technologies, Inc., Madison, Wis.), pyrosequencing techniques (Pyrosequencing, Inc., Westborough, MA) or solid-phase minisequencing (U.S. Patent No. 6,013,431, Suomalainen et al. Mol. Biotechnol. Jun;15(2):123-31, 2000).

In one embodiment the invention is directed to a method of detecting a genetic disorder in a fetus from a plasma sample of a pregnant woman, the method comprising: a) analyzing both maternal and paternal nucleic acid, e.g., DNA for a disease-causing mutation for the single gene disorder; b) if both maternal and paternal nucleic acid, e.g., DNA or RNA, preferably DNA, carry a disease causing mutation for the same disease then isolating nucleic acid, e.g., DNA isolated from blood, plasma or serum of the pregnant mother; c) determining a fetal genotype from the isolated maternal plasma nucleic acid, e.g., DNA using primers corresponding to the paternally identified mutation and performing a mutation-specific primer-extension assay in several replicates, wherein a detection of the paternal mutation in any of the replicate sample is indicative of the presence of the single-gene disorder in the fetus.

The gestational age of the fetus preferably varies from about 7 to about 23 weeks.

The genetic disease detected using the present invention may be any disease wherein a disease causing mutation is known or wherein a genetic polymorphisms associated with or linked to the disease are known. The disease is one caused by mutations in one gene and most preferably, the disease is a recessive single gene disease. The mutations can vary from single nucleotide point mutations to insertions, inversions, and deletions of any number of nucleotides in the genomic DNA. Preferably, the recessive genetic disease is caused by two different mutations wherein one is inherited from the mother and the other from the father. Preferred examples of genetic diseases that can be diagnosed using the method of the present invention include but are not limited to thalassemias, such as beta thalassemias, cystic fibrosis, and congenital adrenal hyperplasia.

DNA isolation from blood, plasma, or serum can be performed using any method known to one skilled in the art. One such method is disclose in Chiu, R. W. K. et al. Clin Chem 47: 1607-1613. (2001). Other suitable methods include, for example TRI REAGENT® BD (Molecular Research Center, Inc.,

Cincinnati, OH), which is a reagent for isolation of DNA from, for example, plasma. TRI REAGENT BD and the single-step method are described, for example, in the US Patent Nos. 4,843,155 and 5,346,994.

Different alleles present in the nucleic acid sample are consequently either amplified using PCR and then differentiated using various differential amplification methods described below, including different primer extension methods. Alternatively, the different alleles are amplified and differentiated simultaneously, for example using the below-described INVARER assay.

In one embodiment, before the primer extension reaction the isolated DNA is amplified using PCR and primers flanking the known mutation site and/or the single nucleotide polymorphism (SNP) site. In one preferred embodiment, primers presented in Table 2 are used to detect the corresponding beta thalassemia mutations shown in the Table 2. In an alternative embodiments, no pre-amplification of the sample is necessary.

In one embodiment, a primer extension reaction is used to detect or "enhance" or "amplify" or "highlight" the different alleles present in the maternal nucleic acid sample. Primer extension reaction can be performed using any protocol for primer extension known to one skilled in the art (see, e.g., Molecular Cloning: A Laboratory Manual, 3rd Ed., Sambrook and Russel, Cold Spring Harbor Laboratory Press, 2001).

For example, methods including complementary DNA (cDNA) arrays (Shalon et al., Genome Research 6(7):639-45, 1996; Bernard et al., Nucleic Acids Research 24(8):1435-42, 1996), solid-phase mini-sequencing technique (U.S. Patent No. 6,013,431, Suomalainen et al. Mol. Biotechnol. Jun;15(2):123-31, 2000), ion-pair high-performance liquid chromatography (Doris et al. J. Chromatogr. A May 8;806(1):47-60, 1998), and 5' nuclease assay or real-time RT-PCR (Holland et al. Proc Natl Acad Sci USA 88: 7276-7280, 1991), or primer extension methods described in the U.S. Patent No. 6,355,433, can be used.

In one embodiment, the primer extension reaction and analysis is performed using PYROSEQUENCING™ (Uppsala, Sweden) which essentially is sequencing by synthesis. A sequencing primer, designed directly next to the nucleic acid differing between the disease-causing mutation and the normal allele or the different SNP alleles is first hybridized to a single stranded, PCR amplified DNA template from the mother, and incubated with the enzymes, DNA polymerase, ATP sulfurylase, luciferase and apyrase, and the substrates, adenosine 5' phosphosulfate (APS) and luciferin. One of four deoxynucleotide triphosphates (dNTP), for example, corresponding to the nucleotide present in the disease-causing allele, is then added to the reaction. DNA polymerase catalyzes the incorporation of the dNTP into the standard DNA strand. Each incorporation event is accompanied by release of pyrophosphate (PPi) in a quantity equimolar to the amount of incorporated nucleotide. Consequently, ATP sulfurylase converts PPi to ATP in the presence of adenosine 5' phosphosulfate. This ATP drives the luciferase-mediated conversion of luciferin to oxyluciferin that generates visible light in amounts that are proportional to the amount of ATP. The light produced in the luciferase-catalyzed reaction is detected by a charge coupled device (CCD) camera and seen as a peak in a PYROGRAM™. Each light signal is proportional to the number of nucleotides incorporated and allows a clear determination of the presence or absence of, for example, the disease causing allele. Thereafter, apyrase, a nucleotide degrading enzyme, continuously degrades unincorporated dNTPs and excess ATP. When degradation is complete, another dNTP is added which corresponds to the dNTP present in for example the selected SNP. Addition of dNTPs is performed one at a time. Deoxyadenosine alfa-thio triphosphate (dATPαS) is used as a substitute for the natural deoxyadenosine triphosphate (dATP) since it is efficiently used by the DNA polymerase, but not recognized by the luciferase. For detailed information about reaction conditions for the PYROSEQUENCING, see, e.g. U. S. Patent No. 6,210, 891.

Another example of the methods useful for detecting the different alleles in the sample isolated from the maternal plasma, serum or blood, is real time PCR. All real-time PCR systems rely upon the detection and quantification of a fluorescent reporter, the signal of which increases in direct proportion to the amount of PCR product in a reaction. Examples of real-time PCR method useful according to the present invention include, TaqMan® and molecular beacons, both of which are hybridization probes relying on fluorescence resonance energy transfer (FRET) for quantitation. TaqMan Probes are oligonucleotides that contain a fluorescent dye, typically on the 5' base, and a quenching dye, typically located on the 3' base. When irradiated, the excited fluorescent dye transfers energy to the nearby quenching dye molecule rather than fluorescing, resulting in a nonfluorescent substrate. TaqMan probes are designed to hybridize to an internal region of a PCR product (ABI 7700 (TaqMan™), Applied BioSystems, Foster City, CA). Accordingly, two different primers, one hybridizing to the disease-causing allele and the other to the selected SNP allele nucleic acid template, are designed. The primers are consequently allowed to hybridize to the corresponding nucleic acids in the real time PCR reaction. During PCR, when the polymerase replicates a template on which a TaqMan probe is bound, the 5' exonuclease activity of the polymerase cleaves the probe. Consequently, this separates the fluorescent and quenching dyes and FRET no longer occurs. Fluorescence increases in each cycle, proportional to the rate of probe cleavage.

Molecular beacons also contain fluorescent and quenching dyes, but FRET only occurs when the quenching dye is directly adjacent to the fluorescent dye. Molecular beacons are designed to adopt a hairpin structure while free in solution, bringing the fluorescent dye and quencher in close proximity. Therefore, for example, two different molecular beacons are designed, one recognizing the disease-causing allele and the other the selected SNP nucleic acid. When the molecular beacons hybridize to the nucleic acids, the fluorescent dye and quencher are separated, FRET does not occur, and the fluorescent dye emits light upon irradiation. Unlike TaqMan probes, molecular beacons are designed to remain intact during the amplification reaction, and must rebind to target in every cycle for signal measurement. TaqMan probes and molecular beacons allow multiple DNA species to be measured in the same sample (multiplex PCR), since fluorescent dyes with different emission spectra may be attached to the different probes, e.g. different dyes are used in making the probes for different disease-causing and SNP alleles. Multiplex PCR also allows internal controls to be co-amplified and permits allele discrimination in single-tube assays. (Ambion Inc, Austin, TX, TechNotes 8(1) - February 2001, Real-time PCR goes prime time).

Yet another method useful according to the present invention for emphasizing or enhancing the difference between the disease-causing and normal allele and the different selected SNP alleles is solid-phase minisequencing (Hultman, et al., 1988, Nucl. Acid. Res., 17, 4937-4946; Syvanen et al., 1990, Genomics, 8, 684-692). In the original reports, the incorporation of a radiolabeled nucleotide was measured and used for analysis of the three-allelic polymorphism of the human apolipoprotein E gene. The method of detection of the variable nucleotide(s) is based on primer extension and incorporation of detectable nucleoside triphosphates in the detection step. By selecting the detection step primers from the region immediately adjacent to the variable nucleotide, this variation can be detected after incorporation of as few as one nucleoside triphosphate. Labelled nucleoside triphosphates matching the variable nucleotide are added and the incorporation of a label into the detection step primer is measured. The detection step primer is annealed to the copies of the target nucleic acid and a solution containing one or more nucleoside triphosphates including at least one labeled or modified nucleoside triphosphate, is added together with a polymerizing agent in conditions favoring primer extension. Either labeled deoxyribonucleoside triphosphates (dNTPs) or chain terminating dideoxyribonucleoside triphosphates (ddNTPs) can be used. The solid-phase mini-sequencing method is described in detail, for example, in the U.S. Patent No. 6,013,431 and in Wartiovaara and Syvanen, Quantitative analysis of human DNA sequences by PCR and solid-phase minisequencing. Mol Biotechnol 2000 Jun; 15(2): 123-131.

Another method to detect the different alleles in the PCR products from the maternal sample is by using fluorescence tagged dNTP/ddNTPs. In addition to use of the fluorescent label in the solid phase mini-sequencing method, a standard nucleic acid sequencing gel can be used to detect the fluorescent label incorporated into the PCR amplification product. A sequencing primer is designed to anneal next to the base differentiating the disease-causing and normal allele or the selected SNP alleles. A primer extension reaction is performed using chain terminating dideoxyribonucleoside triphosphates (ddNTPs) labeled with a fluorescent dye, one label attached to the ddNTP to be added to the standard nucleic acid and another to the ddNTP to be added to the target nucleic acid.

Alternatively, an INVADER® assay can be used (Third Wave Technologies, Inc (Madison, WI)). This assay is generally based upon a structure-specific nuclease activity of a variety of enzymes, which are used to cleave a target-dependent cleavage structure, thereby indicating the presence of specific nucleic acid sequences or specific variations thereof in a sample (see, e.g. U.S. Patent No. 6,458,535). For example, an INVADER® operating system (OS), provides a method for detecting and quantifying DNA and RNA. The INVADER® OS is based on a "perfect match" enzyme-substrate reaction. The INVADER® OS uses proprietary CLEAVASE® enzymes (Third Wave Technologies, Inc (Madison, WI)), which recognize and cut only the specific structure formed during the INVADER® process which structure differs between the different alleles selected for detection, i.e. the disease-causing allele and the normal allele as well as between the different selected SNPs. Unlike the PCR-based methods, the INVADER® OS relies on linear amplification of the signal generated by the INVADER® process, rather than on exponential amplification of the target.

In the INVADER® process, two short DNA probes hybridize to the target to form a structure recognized by the CLEAVASE® enzyme. The enzyme then cuts one of the probes to release a short DNA "flap." Each released flap binds to a fluorescently-labeled probe and forms another cleavage structure. When the CLEAVASE® enzyme cuts the labeled probe, the probe emits a detectable fluorescence signal.

Disease-causing alleles and the SNPs may also be differentiated using allele-specific hybridization followed by a MALDI-TOF-MS detection of the different hybridization products.

In the preferred embodiment, the detection of the enhanced or amplified nucleic acids representing the different alleles is performed using matrix-assisted laser desorption ionization/time-of-flight (MALDI-TOF) mass spectrometric (MS) analysis described in the Examples below. This method differentiates the alleles based on their different mass and can be applied to analyze the products from the various above-described primer-extension methods or the INVADER® process.

It has been shown that during early pregnancy, the median total DNA concentration in maternal plasma is approximately 1000 genome-equivalents per milliliter (Lo, Y.M.D., et al. Am J Hum Genet 62,768-775, 1998). Fetal DNA comprises some 5% of the total DNA in maternal plasma and theoretically, half of which is contributed by the paternally-inherited fraction. Using maternal plasma DNA extraction and PCR protocols as previously described (Lo, Y. M. D. , et al. Am J Hum Genet 62,768-775, 1998) (for example, DNA extraction from about 800 μL of maternal plasma eluted in about 50 μL water, and using about 5 μL of DNA per PCR), each reaction will theoretically contain about 2 copies of the paternally-derived fetal DNA (i.e., in the example above, 1000 x 0.8 x 2.5% x 5/50) and about 76 copies of maternal DNA (i.e., in the example above, 1000 x 0.8 x 95% x 5/50).

The low fractional concentration poses significant demands on the sensitivity and specificity required for the analytical system. In addition, due to the low absolute concentration, fetal DNA fragments in maternal plasma are distributed stochastically (Ding, C & Cantor, C. R. , Proc Natl Acad Sci USA 100,7449-7453, 2003). If analyses were performed in multiple replicates, each replicate would either contain no or only some fetal DNA fragments. The proportion of replicates that does contain fetal DNA fragments is therefore governed by the Poisson distribution. If the experiments were performed at further dilution, for example, using 1 μL of plasma DNA, the amount of maternal DNA per replicate will reduce to one-fifth and for replicates that contain one copy of the fetal-specific paternal allele, the fractional concentration of the fetal fraction will increase from 2.5% (2/80) to 6.25% (1/16), thus, improving the robustness for fetal DNA detection and allelic discrimination.

Therefore, it is important to use several replicates of the analysis. In a preferred embodiment, at least about 15 or more replicates up to about 20, 50, 70 or 100 replicates are used to improve statistical accuracy of the allele determination.

In another embodiment, the invention provides a method wherein single nucleotide polymorphism (SNP) detection is incorporated with the simultaneous detection of the mutated or healthy alleles. SNPs that are associated with either the mutated or normal paternal allele can be used in this embodiment.

The additional assessment of SNPs will also help to eliminate the false-negative results, as a diagnostic result is only regarded as valid if a paternal SNP allele is detected in at least one of the replicates.

Although the MassARRAY system was originally designed for high-throughput SNP detection (Tang K. , et al., Proc Natl Acad Sci USA 96,10016-10020, 1999), the discrimination of paternal and maternal mutant alleles that share the same mutation can be achieved by the detection of SNPs that are linked particularly to the paternal mutation which combination provides an improved method for non-invasive prenatal diagnosis of recessive diseases. The detection of SNPs associated with the paternal normal or healthy allele instead, would, for example, allow the positive exclusion of β-thalassemia (Chiu, R. W. K et al., Clin Chem 48,778-780,2002) in the fetus by using a maternal plasma sample.

## EXAMPLES

### Example 1

The feasibility of assessing fetal gender from maternal plasma using the MassARRAY system is shown herein. Forty-one normal pregnancies comprising 32 male and 9 female fetuses were recruited with informed consent. Ten milliliters of maternal blood was collected. Maternal plasma was obtained and DNA was extracted as previously described (Chiu, R. W. K. et al. Clin Chem 47,1607-1613. (2001)). Fetal gender was determined in the maternal plasma by MassARRAY analysis of the primer extension products of a Y-chromosome-specific PCR and a previously developed real-time quantitative PCR assay (Lo, Y. M. D. et al. Am J Hum Genet 62,768-775 (1998)). For MassARRAY analysis, one microliter of maternal plasma DNA was used in each five microliter PCR reaction. After removing excess dNTPs with a shrimp alkaline phosphatase, base extension reaction was carried out (PCR and extension primer sequences are provided in supplementary table 1). The extension products were analyzed by MALDI-TOF mass spectrometry (SEQUENOM) (Ding, C. & Cantor, C. R. Proc Natl Acad Sci USA 100,7449-7453 (2003)). Fifteen replicates were performed for each sample. The number of replicates was determined by probabilistic calculations based on the Poisson distribution (Fig. 1) to maximize the probability of the positive detection of the fetal DNA molecules in any of the replicates. The fetal gender was reported as male if any of the 15 replicates was positive for the Y-chromosome-specific product. The fetal gender was correctly predicted in all cases. The proportion of replicates with positive Y-chromosome product correlated with that predicted by the theoretical estimates of the Poisson distribution based on the fetal DNA concentration determined by real-time quantitative PCR (Spearman rank correlation R=0.421; p=0.0191) (Fig. 1).

As shown below, the approach allows single nucleotide discrimination between fetal and maternal DNA. We studied pregnancies where the fetus was at risk for β-thalassemia major. The protocol was adopted for maternal plasma analysis of the paternally-inherited fetal-specific *HBB* mutation for the four most common Southeast Asian β-thalassemia mutations. These four mutations included CD 41/42 -CTTT, IVS2 654 (C→T), nt -28 (A→G) and CD 17 (A→T), and they account for about 90% of all β-thalassemia mutations in Southeast Asia (Lau, Y.L. et al. N Engl J Med 336, 1298-1301 (1997)).

Twenty-three couples whose pregnancies were at risk of β-thalassemia major were recruited with institutional consent from the established prenatal diagnostic centers at The Chinese University of Hong Kong, Hong Kong; Chiang Mai University, Thailand; and KK Women's and Children's Hospital, Singapore. Ten milliliters of maternal and paternal blood were collected into EDTA tubes prior to chorionic villus sampling, amniocentesis or cordocentesis. The median gestational age at the time of sampling was about 17 weeks (ranging from about 8 to about 22 weeks).

Parental genotype and mutation analyses were performed according to established diagnostic practices. The 23 pregnancies were fathered by 11 carriers of CD 41/42 -CTTT mutation, 6 carriers of IVS2 654 (C→T) mutation, 1 carrier of nt -28 (A→G) mutation and and 5 individuals who were carriers of the CD 17 (A→T) mutation (Fig. 5, Table 1).

As a control, fetal genotype was determined by molecular analysis of chorionic villi, amniotic fluid or fetal whole blood. The researcher who analyzed the maternal plasma samples was blinded from the fetal genotype result.

Primer extension and MassARRAY analysis were performed essentially in the same manner as the fetal gender experiments with the exception that the fetal targets for detection were the paternally-inherited *HBB* mutations. Four PCR and primer-extension assays were designed and these corresponded to each of the four mutations. The PCR and extension primer sequences are provided in Table 1 (Fig. 6). The assay primers used in a particular sample was selected according to the mutation that the father carried.

By performing 15 replicates for each sample, the presence or absence of the paternal genotype was correctly predicted in 20/23 cases, with the remaining three cases being false negatives (Fig. 5, Table 1). Further analysis with an additional 25-replicate protocol eliminated 2 of the 3 false negative results. Moreover, two of the three false negative cases were performed using samples archived for more than 4 years and had been subjected to repeated freeze-thaw cycles.

The approach of this invention takes advantage of the Poisson distribution of DNA fragments at single molecule concentration detected in replicates to further increase the sensitivity of mass-spectrometric analysis while retaining its high specificity. Using the described example we have shown that the approach allows the determination of the presence or absence of the paternally-inherited fetal mutation in maternal plasma covering the four most common β-thalassemia mutations in Southeast Asians.

Three of the four analyzed mutations are point mutations and thus the results also demonstrate the feasibility of this method to detect even single nucleotide differences at very low fractional concentrations.

The ability to reliably analyze fetal DNA isolated from the maternal plasma offers great opportunity to practically provide non-invasive prenatal diagnosis. Our system using, for example, the MassARRAY approach is automatable with a throughput capability of analyzing more than 1200 samples per day with the about 15-replicate format, thus making the system practical for routine use.

The present approach can be applied to at-risk pregnancies where the maternal and paternal mutations differ. However, the approach can be modified by incorporating the simultaneous detection of single nucleotide polymorphisms (SNPs) that are associated with either the mutated or normal paternal allele. The additional assessment of SNPs will also help to eliminate the false-negative results, as a diagnostic result is only regarded as valid if a paternal SNP allele is detected in at least one of the replicates.

The MassARRAY system was originally designed for high-throughput SNP detection (Tang, K. et al. Proc Natl Acad Sci U S A 96, 10016-10020 (1999)). The discrimination of paternal and maternal mutant alleles that share the same mutation can be achieved by the detection of SNPs that are linked particularly to the paternal mutation. The detection of SNPs associated with the paternal normal or healthy allele instead, also allows the positive exclusion of β-thalassemia (Chiu, R.W.K. et al. Clin Chem 48, 778-780 (2002)). In summary, the analytical and prenatal diagnostic approach of the present invention presents a non-invasive prenatal diagnosis of autosomal recessive diseases including but not limited to the thalassemias, cystic fibrosis and congenital adrenal hyperplasia. This approach can also be applied to the other diagnostic applications of plasma DNA, such as the detection of tumor-derived point mutations in cancer patients (Anker, P. et al. Gastroenterology 112, 1114-1120 (1997)) and donor-derived DNA in the plasma of transplant recipients (Lo, Y.M.D. et al. Lancet 351, 1329-1330 (1998).

## Example 2

In the present study, we evaluated, and show the feasibility of, the use of MS for the discrimination of fetal point mutations in maternal plasma and developed an approach for the reliable exclusion of β-thalassemia mutations in maternal plasma. We further evaluated, and show the feasibility of, the approach for the noninvasive prenatal diagnosis of a mother and father sharing an identical β-thalassemia mutation, a concurrence previously perceived as a challenge for maternal plasma-based prenatal diagnosis for autosomal recessive diseases.

In this example, mass spectrometric analysis of single nucleotide difference in circulating nucleic acids was applied to noninvasive or minimally invasive prenatal diagnosis.

**Patient Recruitment and Sample Collection.** Twelve pregnancies at risk for β-thalassemia major were recruited with informed consent and institutional ethics approval from established prenatal diagnostic centers in Hong Kong, Thailand, Singapore, and Malaysia. Fifty pregnant women seeking second-trimester aneuploidy prenatal diagnosis with subsequent confirmation of a normal fetal karyotype also were recruited. Ten milliliters of maternal and paternal blood was collected into EDTA tubes before amniocentesis, chorionic villus sampling, and cordocentesis. Three milliliters of amniotic fluid also was collected from the normal pregnancies and stored at 4°C until analysis. Parental and fetal genotypes were determined according to established diagnostic practices (Ng, I. S., Ong, J. B., Tan, C. L. & Law, H. Y. (1994) Hum. Genet. 94, 385-388; Sanguansermsri, T., Thanarattanakom, P., Steger, H. F., Tongsong, T., Chanprapaph, P., Wanpirak, C., Siriwatanapa, P., Sirichotiyakul, S. & Flatz, G. (2001) Hemoglobin 25, 19-27). Maternal plasma was harvested by a two-step centrifugation protocol comprised of 10-min centrifugation at 1,600 x g, followed by 10-min centrifugation at 16,000 x g (Chiu, R. W. K., Poon, L. L. M., Lau, T. K., Leung, T. N., Wong, E. M. C. & Lo, Y. M. D. (2001) Clin. Chem. 47, 1607-1613). Maternal plasma DNA was extracted with the QIAamp Blood Kit (Qiagen, Valencia, CA) by following the "blood and body fluid protocol," according to the manufacturer's recommendations. To each column, 800 μl of plasma was applied and eluted into 50 μl of distilled deionized $H_2O$. The plasma DNA samples were stored at -20°C until analysis by a central laboratory.

**Maternal Plasma Analysis.** Paternal allele detection in maternal plasma was performed by using the MassARRAY system (Sequenom). The MassARRAY system is a matrix-assisted laser desorption ionization/time-of-flight MS system designed for the detection of primer-extended PCR products (Tang, K., Fu, D. J., Julien, D., Braun, A., Cantor, C. R. & Koster, H. (1999) Proc. Natl. Acad. Sci. USA 96, 10016-10020). The maternal plasma MS analyses were performed blindly without knowledge of the fetal genotype. Two analytical protocols were evaluated, including the standard Homogenous MassEXTEND protocol provided by Sequenom and a newly developed protocol, termed single allele base extension reaction (SABER) (Fig. 3). Both protocols involved PCR amplification of the paternally inherited fetal allele and the maternal background alleles from maternal plasma, followed by a base extension reaction before MS analysis. The SABER protocol involves a different base extension step, which is restricted to the allele of interest, and confers theoretical improvements in the detection sensitivity.

**PCR Amplification.** All DNA oligonucleotides were purchased from Integrated DNA Technologies (Coralville, IA). HotStar *Taq* Polymerase (Qiagen) was used for all PCRs. Five microliters of plasma DNA was added to each 10-μl PCR. PCR primers (Fig. 6, Table 2) were used at a 200 nM final concentration. The PCR condition was 95°C for 15 min for hot start, followed by denaturing at 94°C for 20 sec, annealing at 56°C for 30 sec, extension at 72°C for 1 min for 45 cycles, and final incubation at 72°C for 3 min. Five microliters of PCR products was treated with shrimp alkaline phosphatase (Sequenom) for 20 min at 37°C to remove excess dNTPs, as described in Ding, C. & Cantor, C. R. (2003) Proc. Natl. Acad. Sci. USA 100, 7449-7453.

**Standard Base Extension and SABER.** Thermosequenase (Sequenom) was used for the base extension reactions. In the standard protocol, conventional base extension was carried out whereby both alleles interrogated by the base extension primer were extended by adding a mixture of 2',3'-dideoxynucleoside triphosphates and dNTPs (Fig 6, Table 2 and Fig. 3). In contrast, primer extension in the SABER protocol was restricted to the fetal-specific allele of interest by the addition of a single species of dideoxynucleoside triphosphate without any dNTP (Fig. 6, Table 2 and Fig. 3). Five microliters of PCR products was used in 9-$\mu$l reactions in both protocols. The reaction condition was 94°C for 2 min, followed by 94°C for 5 sec, 52°C for 5 sec, and 72°C for 5 sec for 40 cycles. All reactions were carried out in a GeneAmp PCR system 9700 thermal cycler (Applied Biosystems). The final base extension products were analyzed by MS as described in Ding, C. & Cantor, C. R. (2003) Proc. Natl. Acad. Sci. USA 100, 7449-7453. Briefly, the final base extension products were treated with the Spectro-CLEAN (Sequenom) resin to remove salts in the reaction buffer. We dispensed ≈10 nl of reaction solution onto a 384-format SpectroCHIP (Sequenom) prespotted with a matrix of 3-hydroxypicolinic acid by using a SpectroPoint (Sequenom) nanodispenser. A modified Biflex matrix-assisted laser desorption ionization/time-of-flight MS (Bruker, Billerica, MA) was used for data acquisitions from the SpectroCHIP. The expected molecular weights of all relevant peaks were calculated before the analysis and identified from the mass spectrum. All analyses were performed in triplicate.

**Fetal-Specific Single-Nucleotide Polymorphism (SNP) Detection from Maternal Plasma.** The feasibility of using the MassARRAY system to discriminate and detect single-nucleotide differences between fetal and maternal DNA in maternal plasma was first assessed by the detection of paternally inherited SNPs. The maternal and fetal genotypes for 11 SNPs on chromosome 11p were determined in normal pregnancies by using maternal genomic DNA and amniotic fluid samples. The most informative SNP, rs2187610 (SNP database, www.ncbi.rlm.nih.gov), was selected for further analysis. This SNP is located 1.3 kb downstream of the *HBB* locus.

**Fetal-Specific β-Thalassemia Mutation Detection from Maternal Plasma.** MassARRAY assays (Fig. 6, Table 2) were designed for maternal plasma analysis of the four most common β-thalassemia mutations in Southeast Asia, CD 41/42 -CTTT, IVS2 654 (C 3 T), nt -28 (A →G), and CD 17 (A →T) (Lau, Y. L.; Chan, L. C., Chan, Y. Y., Ha, S. Y., Yeung, C. Y., Waye, J. S. & Chui, D. H. (1997) N. Engl. J. Med. 336, 1298-1301; Liang, R., Liang, S., Jiang, N. H., Wen, X. J., Zhao, J. B., Nechtman, J. F., Stoming, T. A. & Huisman, T. H. (1994) Br. J. Haematol. 86, 351-354). Paternal mutation detection in maternal plasma was determined by using both protocols. For each sample, the mutation-specific assay was selected according to the mutation that the father carried.

**Fetal Haplotype Detection from Maternal Plasma.** The parental genotypes at the SNP locus, rs2187610, were determined for the pregnancies at risk for β-thalassemia major. For parents who were found to be informative for the SNP, the linkage between the paternal *HBB* mutant with the SNP alleles at ras2187610 was determined. Haplotype analysis was determined by using a method on parental genomic DNA described in Ding, C. & Cantor, C. R. (2003) Proc. Natl. Acad. Sci. USA 100, 7449-7453. The ability to detect the paternal SNP linked to the mutant *HBB* allele in maternal plasma was determined by using the SABER protocol.

**Fetal-Specific SNP Allele Discrimination in Maternal Plasma.** The SNP rs2187610 is a C/G polymorphism. Among the 50 normal pregnancies, 16 pregnant women had the CC genotype. The fetal genotypes were CC and GC in 10 and 6 of these pregnancies, respectively. MassARRAY assays were designed to detect the paternally inherited fetal-specific G allele in maternal plasma (Fig. 6, Table 2). The presence or absence of the G allele in maternal plasma was concordant between the standard and SABER protocols, and these results were completely concordant with amniotic fluid analyses.

**Paternally Inherited β-thalassemia Point Mutation Detection and Exclusion in Maternal Plasma.** Among the 12 recruited pregnancies at risk for β-thalassemia major, 11 pregnancies involved couples in which the father and mother carried different β-thalassemia mutations (Fig. 7, Table 3). Assays were designed to interrogate the four β-thalassemia mutations in maternal plasma, three of which were point mutations. The results are shown in (Fig. 7, Table 3). Detection of the paternal mutation in maternal plasma by using the SABER protocol was completely concordant with the fetal genotype determined by amniotic fluid, chorionic villus, or fetal blood analyses, whereas the standard protocol revealed two false-negative results (cases 5 and 9). Representative MS tracings for the analyses are shown in Fig. 4.

**Noninvasive Fetal Haplotyping.** SNP analysis for the at-risk pregnancies revealed three informative couples (cases 3, 11, and 12), including the parents sharing an identical β-thalassemia mutation, whereby the maternal and paternal SNP genotypes were nonidentical. Results of the haplotype analysis are shown in Fig. 8, Table 4. The paternal mutant allele was linked to the *G* allele at rs2187610 for the three cases. Maternal plasma analysis for the paternal G allele was completely concordant with the expected fetal genotype.

The reliable discrimination of subtle (e.g., single base) differences between fetal and maternal DNA in maternal plasma has hitherto been a technical challenge (Nasis, O., Thompson, S., Hong, T., Sherwood, M., Radcliffe, S., Jackson, L. & Otevrel, T. (2004) Clin. Chem. 50, 694-701). In this study, we took advantage of the analytical specificity conferred by a base extension reaction and the sensitivity of MS analysis. The SABER protocol is theoretically more sensitive than the standard protocol. First, in contrast to the standard protocol in which all relevant alleles are used as the templates for the base extension reaction, SABER involves the extension of a single nucleotide for the allele of interest only (Fig. 3). Thus, for fetal DNA analysis in maternal plasma, the SABER assays were designed so that the base extension is devoted only to the extension of the fetal-specific allele for the single discriminatory nucleotide from the maternal one. Furthermore, the matrix-assisted laser desorption ionization/time-of-flight MS has a dynamic range of ≈100-fold. Because the paternal-specific fetal allele exists at ≈3-6% in total maternal plasma DNA, its corresponding peak in the mass spectrum is often dwarfed by the background peak when analyzed by the standard protocol (Figs. 3 and 4). In contrast, the SABER method only extends the intended paternal-specific fetal allele so that the background allele peak is not produced, resulting in more robust detection (Figs. 3 and 4). The theoretical advantages of SABER over the standard method are realized in our analyses as evident by the false-negative results for the latter protocol.

The reliability of the SABER assays for single-nucleotide discrimination between circulating fetal and maternal DNA has been illustrated by the maternal plasma detection of fetal β-thalassemia point mutations and SNPs. The ability to robustly analyze fetal-specific SNPs in maternal plasma is a useful adjunct procedure for maternal-plasma fetal DNA analysis as a safeguard against the possibility of false-negative detection due to fetal DNA degradation, DNA extraction failures, or PCR allele dropout. Such a safeguard mechanism has been advocated by several workers in the routine performance of maternal plasma analysis for the noninvasive prenatal assessment of fetal rhesus D status (van der Schoot, C. E., Tax, G. H., Rijnders, R. J., de Haas, M. & Christiaens, G. C. (2003) Transfusion Med. Rev. 17, 31-44; Zhong, X. Y., Holzgreve, W. & Hahn, S. (2001) Swiss Med. Wkly. 131, 70-74; Avent, N. D., Finning, K. M., Martin, P. G. & Soothill, P. W. (2000) Vox Sanguinis 78, 155-162). Initially, the detection of Y-chromosome sequences in maternal plasma had been adopted to confirm cases that tested negative for *RHD* (Finning, K. M., Martin, P. G., Soothill, P. W. & Avent, N. D. (2002) Transfusion 42, 1079-1085; van der Schoot, C. E., Tax, G. H., Rijnders, R. J., de Haas, M. & Christiaens, G. C. (2003) Transfusion Med. Rev. 17, 31-44). Because of the inherent restriction of Y-chromosome detection to only male fetuses, fetal-specific internal controls based on panels of insertion/deletion polymorphisms had been developed (Avent, N. D., Finning, K. M., Martin, P. G. & Soothill, P. W. (2000) Vox Sanguinis 78, 155-162). The adoption of the insertion/deletion panel reflects the lack of robust methods for fetal SNP detection in the past. Hence, with the availability of a reliable MS method for fetal SNP detection in maternal plasma, the number of potential gender-independent internal control targets for circulating fetal DNA detection has increased substantially.

A more important implication of the ability to analyze circulating fetal SNPs lies in its immediate relevance to fetal haplotype analysis from maternal plasma. Noninvasive fetal haplotyping could be achieved by means of analyzing polymorphisms linked to a mutated locus. As demonstrated in case 12, haplotype analysis between the *HBB* locus and a linked polymorphism allowed the noninvasive prenatal exclusion of β-thalassemia major, despite the presence of the same *HBB* mutation in both parents, which had previously not been believed practical. (Chiu, R. W. K., Lau, T. K., Leung, T. N., Chow, K. C. K., Chui, D.

H. K. & Lo, Y. M. D. (2002) Lancet 360, 998-1000). The haplotype approach is also applicable to maternal plasma detection of a fetal SNP allele linked to the paternal nonmutant allele. The positive detection of such an allele would allow for the positive prenatal exclusion of β-thalassemia major noninvasively (Chiu, R. W. K., Lau, T. K., Cheung, P. T., Gong, Z. Q., Leung, T. N. & Lo, Y. M. D. (2002) Clin. Chem. 48, 778-780; Bianchi, D. W. (2002) Clin. Chem. 48, 689-690).

Additional SNP markers surrounding the *HBB* locus can be easily assessed by a person skilled in the art. For example, an SNP panel could be assembled so that the noninvasive prenatal diagnosis could be applied to a larger proportion of pregnancies at risk for P-thalassemia. The four mutations investigated in this study account for 90% of all β-thalassemia mutations in Southeast Asia (Lau, Y. L., Chan, L. C., Chan, Y. Y., Ha, S. Y., Yeung, C. Y., Waye, J. S. & Chui, D. H. (1997) N. Engl. J. Med. 336, 1298-1301; Liang, R., Liang, S., Jiang, N. H., Wen, X. J., Zhao, J. B., Nechtman, J. F., Stoming, T. A. & Huisman, T. H. (1994) Br. J. Haematol. 86, 351-354). The present approach can easily be applied to all pregnancies in which the father is a carrier of one of the four mutations and thus has much potential for routine adoption. An invasive prenatal diagnostic procedure could be avoided in 50% of these pregnancies in which the lack of inheritance of the paternal mutation by the fetus is confirmed by maternal plasma analysis.

This study shows technological advancements in circulating fetal DNA analysis, and that we have developed robust system for single-nucleotide discrimination among circulating DNA species. The MassARRAY approach is automatable with a capacity to analyze >2,000 samples per day in triplicate, thus making the system practical for routine use. The MS system is potentially to many other areas of fetal DNA detection, namely the prenatal diagnosis of other single-gene disorders and the quantification of fetal DNA in maternal plasma (Ding, C. & Cantor, C. R. (2003) Proc. Natl. Acad. Sci. USA 100, 3059-3064). Quantitative aberrations in circulating fetal DNA concentrations have been demonstrated for fetal chromosomal aneuploidies (Lo, Y. M. D., Lau, T. K., Zhang, J., Leung, T. N., Chang, A. M., Hjelm, N. M., Elmes, R. S. & Bianchi, D. W. (1999) Clin. Chem. 45, 1747-1751; Zhong, X. Y., Burk, M. R., Troeger, C., Jackson, L. R., Holzgreve, W. & Hahn, S. (2000) Prenatal Diagn. 20, 795-798), preeclampsia (Lo, Y. M. D., Leung, T. N., Tein, M. S., Sargent, I. L., Zhang, J., Lau, T. K., Haines, C. J. & Redman, C. W. (1999) Clin. Chem. 45, 184-188; Zhong, X. Y., Laivuori, H., Livingston, J. C., Ylikorkala, O., Sibai, B. M., Holzgreve, W. & Hahn, S. (2001) Am. J. Obstet. Gynecol. 184, 414-419), preterm labor (Leung, T. N., Zhang, J., Lau, T. K., Hjelm, N. M. & Lo, Y. M. D. (1998) Lancet 352, 1904-1905), and many other pregnancy-associated complications. Quantitative analysis of circulating fetal DNA has been reliant on the detection of Y-chromosome sequences because of the lack of gender-independent fetal-specific markers. However, this hurdle can be overcome by the adoption of MS quantification of fetal SNPs in maternal plasma. Both the MS approach and the gender-independent fetal SNP assays can be extended to the study of fetal DNA in other maternal bodily fluids such as urine (Botezatu, I., Serdyuk, O., Potapova, G., Shelepov, V., Alechina, R., Molyaka, Y., Ananev, V., Bazin, I., Garin, A., Narimanov, M., et al. (2000) Clin. Chem. 46, 1078-1084) and cerebrospinal fluid (Angert, R. M., Leshane, E. S., Yarnell, R. W., Johnson, K. L. & Bianchi, D. W. (2004) Am. J. Obstet. Gynecol. 190, 1087-1090) or the phenomenon of cellular microchimerism (Bianchi, D. W. & Romero, R. (2003) J. Maternal Fetal Neonatal Med. 14, 123-129; Nelson, J. L. (2001) Lancet 358, 2011-2012; Lo, Y. M. D., Lo, E. S. , Watson, N. , Noakes, L. , Sargent, I. L. , Thilaganathan, B. & Wainscoat, J. S. (1996) Blood 88, 4390-4395), all of which also have been previously studied by means of the detection of Y-chromosome sequences (Lo, Y. M. D., Patel, P., Wainscoat, J. S., Sampietro, M. , Gillmer, M. D. & Fleming, K. A. (1989) Lancet 2, 1363-1365 ; Lo, Y. M. D., Patel, P., Wainscoat, J. S. & Fleming, K. A. (1990) Lancet 335, 724 (lett.)).

In addition to fetal DNA sequences, the MS SABER approach can also be extended to other areas of circulating nucleic acid analysis, including circulating tumor-specific DNA, such as Epstein-Barr virus DNA in nasopharyngeal carcinoma patients (Lo, Y. M. D., Chan, L. Y. S., Lo, K. W., Leung, S. F., Zhang, J., Chan, A. T. C., Lee, J. C. , Hjelm, N. M. , Johnson, P. J. & Huang, D. P. (1999) Cancer Res. 59,1188-1191), KRAS point mutations (Anker, P., Lefort, F. , Vasioukhin, V. , Lyautey, J. , Lederrey, C., Chen, X. Q., Stroun, M. , Mulcahy, H. E. & Farthing, M. J. (1997) Gastroenterology 112,1114-1120; Sorenson, G. D. (2000) Ann. N. Y. Acad. Sci.. 906, 13-16), and donor-specific DNA in transplant recipients (Lo, Y. M. D. , Tein, M. S. , Pang, C. C. , Yeung, C. K., Tong, K. L. & Hjelm, N. M. (1998) Lancet 351,1329-1330). Therefore, we believe that MS will play an increasingly important role in the future research and application of circulating nucleic acids.

## Claims

1. A method of determining a single gene disorder in a fetus from a plasma, whole blood or serum sample of a pregnant mother, the method comprising:

   (a) analyzing nucleic acid samples isolated from the pregnant mother and a father for a disease-causing mutation for a single gene disorder or a single nucleotide polymorphism associated with a disease-causing mutation;
   (b) isolating nucleic acid from blood, plasma, or serum of the pregnant mother;
   (c) determining a fetal genotype from the nucleic acid isolated in step (b) using primers corresponding to a disease-causing mutation allele or mutation-associated allele containing a single nucleotide polymorphism identified in the nucleic acid from the father in step (a) and differentially amplifying the alleles from the isolated nucleic acid sample of step (b) in replicates using a mutation-specific primer-extension assay and detecting the amplified products, wherein a detection of a paternal mutation in any of the replicate samples is indicative of the presence of the single-gene disorder in the fetus;

   wherein the primer-extension assay is performed on at least 15 and up to 1000 replicates in which the proportion of replicates that contain fetal DNA fragments is governed by Poisson distribution.

2. The method of claim 1, wherein the primer-extension assay is performed on at least 15 and up to 100 replicates.

3. The method of claim 1, wherein the mutation-specific primer-extension assay comprises adding a single dideoxynucleotide corresponding to the allele that causes the single gene disorder, without any dNTP.

4. The method of any one of the preceding claims, wherein analyzing products of the primer-extension assay is performed using mass spectrometry.

5. The method of claim 1, wherein the differential amplification is followed by detection using a MassARRAY system.

6. The method of any one of the preceding claims, wherein the single gene disorder is an autosomal recessive disease.

7. The method of claim 6, wherein the autosomal recessive disease is selected from beta thalassemia, cystic fibrosis and congenital adrenal hyperplasia.

8. The method of claim 7, wherein the beta thalassemia is caused by mutations selected from the group consisting of CD 41/42 -CTTT; IVS2 654 (C→T); nucleotide -28 (A→G); and CD 17 (A→T).

## Patentansprüche

1. Verfahren zur Bestimmung einer Einzelgen-Erbkrankheit in einem Fötus aus einer Plasma-, Vollblut- oder Serumprobe einer schwangeren Mutter, das Verfahren umfassend

(a) Analysieren von Nukleinsäureproben, isoliert aus der schwangeren Mutter und einem Vater, auf eine erkrankungsverursachende Mutation für eine Einzelgen-Erbkrankheit oder einen einzelnen Nukleotid-Polymorphismus in Zusammenhang mit einer erkrankungsverursachenden Mutation;

(b) Isolieren von Nukleinsäure aus Blut, Plasma oder Serum der schwangeren Mutter;

(c) Bestimmen eines fötalen Genotyps aus der in Schritt (b) isolierten Nukleinsäure mit Hilfe von Primern, entsprechend einem Allel einer erkrankungsverursachenden Mutation oder einem Allel in Zusammenhang mit einer Mutation, enthaltend einen einzelnen Nukleotid-Polymorphismus, identifiziert in der Nukleinsäure vom Vater in Schritt (a), und differentielle Amplifikation der Allele aus der isolierten Nukleinsäurenprobe aus Schritt (b) in Replikaten mit Hilfe eines mutationsspezifischen Primer-Extensionsassays und Erfassen der amplifizierten Produkte, wobei eine Erfassung einer väterlichen Mutation in irgendeiner der Replikatproben das Vorliegen der Einzelgen-Erbkrankheit im Fötus anzeigt;

wobei der Primer-Extensionsassay an mindestens 15 und bis zu 1000 Replikaten ausgeführt wird, in denen der Anteil Replikate, die fötale DNA-Fragmente enthalten, durch die Poisson-Verteilung bestimmt wird.

**2.** Verfahren gemäß Anspruch 1, wobei der Primer-Extensionsassay an mindestens 15 und bis zu 100 Replikaten ausgeführt wird.

**3.** Verfahren gemäß Anspruch 1, wobei der mutationsspezifische Primer-Extensionsassay umfasst Zusetzen eines einzelnen Dideoxynukleotids, entsprechend dem Allel, das die Einzelgen-Erbkrankheit auslöst, ohne dNTP.

**4.** Verfahren gemäß irgendeinem der vorhergehenden Ansprüche, wobei Analysieren von Produkten des Primer-Extensionsassays mit Hilfe von Massenspektrometrie ausgeführt wird.

**5.** Verfahren gemäß Anspruch 1, wobei die differentielle Amplifikation von einem Erfassen mit Hilfe eines MassARRAY-Systems gefolgt wird.

**6.** Verfahren gemäß irgendeinem der vorhergehenden Ansprüche, wobei die Einzelgen-Erbkrankheit eine autosomal-rezessive Krankheit ist.

**7.** Verfahren gemäß Anspruch 6, wobei die autosomal-rezessive Krankheit ausgewählt ist aus β-Thalassämie, Mukoviszidose und adrenogenitalem Syndrom.

**8.** Verfahren gemäß Anspruch 7, wobei die β-Thalassämie ausgelöst wird durch Mutationen, ausgewählt aus der Gruppe CD 41/42-CTTT; IVS2 654 (C→T); Nukleotid-28 (A→G); und CD 17 (A→T).

**Revendications**

**1.** Un procédé de détermination d'une affection monogénique chez un foetus à partir d'un échantillon de plasma, de sang total ou de sérum d'une femme enceinte, le procédé comprenant :

(a) l'analyse d'échantillons d'acides nucléiques isolés de la femme enceinte et d'un père pour une mutation pouvant entraîner une affection monogénique ou un polymorphisme mononucléotidique associé à une mutation pouvant entraîner la maladie ;

(b) l'isolement de l'acide nucléique du sang, du plasma ou du sérum de la femme enceinte ;

(c) la détermination d'un génotype foetal à partir de l'acide nucléique isolé à l'étape (b) en utilisant des amorces correspondant à un allèle de mutation ou à un allèle associé à une mutation contenant un polymorphisme mononucléotidique identifié dans l'acide nucléique du père à l'étape (a) et l'amplification de manière différentielle des allèles de l'échantillon d'acide nucléique isolés à l'étape (b) en réplicats, en utilisant un essai d'extension d'amorce spécifique de la mutation et en détectant des produits amplifiés, dans lequel la détection d'une mutation paternelle dans l'un des échantillons répliqués est indicative de la présence de l'affection monogénique chez le foetus ;

dans lequel l'essai d'extension d'amorce est effectué sur au moins 15 et jusqu'à 1000 réplicats dans lesquels la proportion de réplicats qui contiennent des fragments d'ADN foetal est régie par la distribution de Poisson.

**2.** Le procédé selon la revendication 1, dans lequel l'essai d'extension d'amorce est effectué sur au moins 15 et jusqu'à 100 réplicats.

**3.** Le procédé selon la revendication 1, dans lequel l'essai d'extension d'amorce spécifique de la mutation comprend l'ajout d'un didésoxynucléotide simple correspondant à l'allèle qui provoque l'affection monogénique, sans aucun dNTP.

**4.** Le procédé selon l'une quelconque des revendications précédentes, dans lequel l'analyse des produits de l'essai d'extension d'amorce est effectuée par spectrométrie de masse.

5. Le procédé selon la revendication 1, dans lequel l'amplification différentielle est suivie par une détection en utilisant un système MassARRAY.

6. Le procédé selon l'une quelconque des revendications précédentes, dans lequel l'affection monogénique est une maladie autosomique récessive.

7. Le procédé selon la revendication 6, dans lequel la maladie autosomique récessive est choisie parmi la bêta-thalassémie, la fibrose kystique et l'hyperplasie surrénale congénitale.

8. Le procédé selon la revendication 7, dans lequel la bêta-thalassémie est causée par des mutations sélectionnées dans le groupe se composant de CD 41/42-CTTT ; IVS2 654 (C→T) ; nucléotide-28 (A→G) ; et CD 17 (A→T).

**FIG. 1**

FIG. 2A

*FIG. 2B*

*FIG. 3*

*FIG. 4A*

*FIG. 4B*

*FIG. 4C*

*FIG. 4D*

EP 1 664 077 B1

| Case | HBB Mutation[a] | | | | Fetal genotype[b] | No. of positive replicates[c] | Gestational age (weeks) |
|---|---|---|---|---|---|---|---|
| | CD 41/42 (-CTTT) | IVS2 654 (C→T) | nt −28 (A→G) | CD 17 (A→T) | | | |
| 1 | F | | | | F/M | 6 | 14 |
| 2 | M | F | | | F/* | 4 | 12 |
| 3 | M | F | | | F/* | 4 | 16 |
| 4 | F | M | | | */* | 0 | 10 |
| 5 | | F | | M | */* | 0 | 18 |
| 6 | F | | | M | */* | 0 | 21 |
| 7 | M | F | | | F/* | 3 | 17 |
| 8 | F | M | | | F/* | 7 | 13 |
| 9 | F | | M | | F/M | 3 | 18 |
| 10 | M | F | | | */* | 0 | 11 |
| 11 | M | | F | | F/M | 7 | 16 |
| 12 | M | | | F | */* | 0 | 22 |
| 13 | F | M | | | */M | 0 | 12 |
| 14 | F | | | M | F/M | 3 | 18 |
| 15 | M | F | | | F/M | 0 | 8 |
| 16 | F | M | | | F/M | 0 | 12 |
| 17 | F | | M | | */M | 0 | 12 |
| 18 | F | | M | | F/M | 2 | 19 |
| 19 | | | | F | F/* | 6 | 17 |
| 20 | | | | F | F/* | 0 | 19 |
| 21 | | | | F | F/M | 3 | 19 |
| 22 | | | | F | */* | 0 | 19 |
| 23 | F | | | M | F/* | 10 | 20 |

FIG. 5

| Mutation | CD 41/42 (-CTTT) | IVS2 654 (C→T) | nt—28 (A→G) | CD 17 (A→T) | rs2187610 |
|---|---|---|---|---|---|
| PCR primer 1 | 5'-ACGTTGGATGTAACAGCATCAGGAGTGGAC-3' (SEQ ID NO:1) | 5'-ACGTTGGATGTAACAGTGATAATTTCTGGG-3' (SEQ ID NO:4) | 5'-ACGTTGGATGTAGGGTTGGCCAATCTACTC-3' (SEQ ID NO:7) | 5'-ACGTTGGATGTCACCACCAACTTCATCCAC-3' (SEQ ID NO:10) | 5'-ACGTTGGATGATGCCATTTCATGGTTACC-3' (SEQ ID NO:13) |
| PCR primer 2 | 5'-ACGTTGGATGCTATTTTCCCACCCTTAGGC-3' (SEQ ID NO:2) | 5'-ACGTTGGATGGAAACCTCTTACATCAGTTAC-3' (SEQ ID NO:5) | 5'-ACGTTGGATGAGCAATAGATGGCTCTGCCC-3' (SEQ ID NO:8) | 5'-ACGTTGGATGTCAAACAGACACCATGGTGC-3' (SEQ ID NO:11) | 5'-ACGTTGGATGGAAGTGAGGCTACATCAAAC-3' (SEQ ID NO:14) |
|  |  |  | Standard protocol |  |  |
| Extension primer | 5'-GATCCCCAAAGGACTCAA-3' (SEQ ID NO:3) | 5'-TGATAATTTCTGGGTTAAGG-3' (SEQ ID NO:6) | 5'-AGCCAGGGCTGGGCATA-3' (SEQ ID NO:9) | 5'-TTCATCCACGTTCACCT-3' (SEQ ID NO:12) | 5'-ACCTTTCATTTGTTCATTGTTTT-3' (SEQ ID NO:15) |
| Terminator mix* | CGT | AC | AC | CGT | ACT |
| Expected molecular weight of extended nonmutant allele | 6,088 | 6,475 | 5,558 | 5,345 | 7,225 (G allele) |
| Expected molecular weight of extended mutant allele | 5,735 | 6,804 | 5,887 | 5,683 | 7,569 (C allele) |
|  |  |  | SABER protocol |  |  |
| Extension primer | 5'-GATCCCCAAAGGACTCAA-3' (SEQ ID NO:16) | 5'-ATATGCAGAAATATTGCTATT-3' (SEQ ID NO:17) | 5'-GATGGCTCTGCCCTGACTT-3' (SEQ ID NO:18) | 5'-TTACTGCCCTGTGGGGC-3' (SEQ ID NO:19) | 5'-ACCTTTCATTTGTTCATTGTTTT-3' (SEQ ID NO:20) |
| Terminator | ddCTP | ddATP | ddCTP | ddTTP | ddCTP |
| Expected molecular weight of nonextended primer | 5,462 | 6,443 | 5,771 | 5,193 | 6,952 |
| Expected molecular weight of extended allele | 5,735 | 6,741 | 6,044 | 5,482 | 7,225 (G allele) |

## FIG. 6

| | Genotype for SNP rs2187610 | | Paternal haplotype analysis[†] | | Maternal plasma SABER analysis | | |
|---|---|---|---|---|---|---|---|
| Case | Mother | Father | *HBB* mutant allele | *HBB* wild-type allele | SNP G allele | Paternal *HBB* mutation | Fetal *HBB* genotype[†] |
| 3 | CC | GC | G | C | Neg | Neg | */* |
| 11 | CC | GC | G | C | Pos | Pos | F/* |
| 12 | CC | GC | G | C | Neg | N.A. | */* |

*FIG. 7*

| | HBB mutation | | | | Maternal plasma analysis | | | |
| Case | CD 41/42 (-CTTT) | IVS2 654 (C→T) | nt—28 (A→G) | CD 17 (A→T) | Standard protocol | SABER | Fetal genotype[†] | Weeks gestation |
|---|---|---|---|---|---|---|---|---|
| 1 | F | M | — | — | Neg | Neg | */* | 11 |
| 2 | — | F | — | M | Neg | Neg | */* | 18 |
| 3 | F | — | — | M | Neg | Neg | */* | 21 |
| 4 | M | F | — | — | Pos | Pos | F/* | 18 |
| 5 | M | — | F | — | Neg | Pos | F/M | 17 |
| 6 | F | M | — | — | Pos | Pos | F/* | 11 |
| 7 | F | M | — | — | Pos | Pos | F/* | 14 |
| 8 | F | — | — | — | Neg | Neg | */* | 7 |
| 9 | — | — | — | F | Neg | Pos | F/* | 12 |
| 10 | M | F | — | — | Neg | Neg | */* | 17 |
| 11 | F | — | — | — | Pos | Pos | F/* | 20 |
| 12 | M & F | — | — | — | N.A. | N.A. | */* | 18 |

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6013431 A
- US 4843155 A
- US 5346994 A
- US 6355433 B
- US 6210891 B
- US 6458535 B

### Non-patent literature cited in the description

- **LO, Y. M. D. et al.** *Lancet,* 1997, vol. 350, 485-487
- **LO, Y. M. D. et al.** *Am J Hum Genet,* 1998, vol. 62, 768-775
- **COSTA, J.M. ; BENACHI, A. ; GAUTIER, E.** *N Engl J Med,* 2002, vol. 346, 1502
- **LO, Y.M.D. et al.** *N Engl JMed,* 1998, vol. 339, 1734-1738
- **CHIU, R.W.K. ; LO, Y.M.D.** *Expert Rev Mol Diagn,* 2002, vol. 2, 32-40
- **SEKIZAWA, A. ; KONDO, T. ; IWASAKI, M. ; WATANABE, A. ; JIMBO, M. ; SAITO, H. ; OKAI, T.** *Clin. Chem.,* 2001, vol. 47, 1856-1858
- **FINNING, K. M. ; MARTIN, P. G. ; SOOTHILL, P. W. ; AVENT, N. D.** *Transfusion,* 2002, vol. 42, 1079-1085
- **COSTA, J. M. ; BENACHI, A. ; GAUTIER, E. ; JOUANNIC, J. M. ; ERNAULT, P. ; DUMEZ, Y.** *Prenatal Diagn.,* 2001, vol. 21, 1070-1074
- **RIJNDERS, R. J. ; CHRISTIAENS, G. C. ; BOSSERS, B. ; VAN DER SMAGT, J. J. ; VAN DER SCHOOT, C. E. ; DE HAAS, M.** *Obstet. Gynecol.,* 2004, vol. 103, 157-164
- **LO Y et al.** *Am. J. Hum. Genet.,* 1998, vol. 62, 768-775
- **HEID et al.** *Genome Res.,* 1996, vol. 6, 986-994
- **NASIS, O. ; THOMPSON, S. ; HONG, T. ; SHERWOOD, M. ; RADCLIFFE, S. ; JACKSON, L. ; OTEVREL, T.** *Clin. Chem.,* 2004, vol. 50, 694-701
- **SAITO, H. ; SEKIZAWA, A. ; MORIMOTO, T. ; SUZUKI, M. ; YANAIHARA, T.** *Lancet,* 2000, vol. 356, 1170
- **GONZALEZ-GONZALEZ, M. C. ; TRUJILLO, M. J. ; RODRIGUEZ DE ALBA, M. ; RAMOS, C.** *Neurology,* 2003, vol. 60, 1214-1215
- **GONZALEZ-GONZALEZ, M. C. ; GARCIA-HOYOS, M. ; TRUJILLO, M. J. ; RODRIGUEZ DE ALBA, M. ; LORDA-SANCHEZ, I. ; DIAZ-RECASENS, J. ; GALLARDO, E. ; AYUSO, C. ; RAMOS, C.** *Prenatal Diagn.,* 2002, vol. 22, 946-948
- **FUCHAROEN, G. ; TUNGWIWAT, W. ; RATANASIRI, T. ; SANCHAISURIYA, K. ; FUCHAROEN, S.** *Prenatal Diagn.,* 2003, vol. 23, 393-396
- **LO, Y. M. D.** *J. Pathol.,* 1994, vol. 174, 1-6
- **CHIU, R.W.K. et al.** *Clin Chem,* 2002, vol. 48, 778-780
- **WEATHERALL, D.J. ; CLEGG, J.B.** *Bull World Health Organ,* 2001, vol. 79, 704-712
- **WEATHERALL, D. J.** *BMJ314,* 1997, 1675-1678
- The hemoglobinopathies. **WEATHERALL et al.** The Metabolic and Molecular Bases of Inherited Disease. McGraw-Hill, 1995, 3417-3484
- **MODEL et al.** Survival in beta-thalassaemia major in the UK: data from the UK Thalassaemia Register. *Lancet,* 2000, vol. 355, 2051-2052
- **STEINBERG, M. H. ; ADAMS, J. G.** Thalassemia: recent insights into molecular mechanisms. *Am. J. Hemat.,* 1982, vol. 12, 81-92
- **CHEUNG, M.C. ; GOLDBERG, J.D. ; KAN, Y.W.** *Nat Genet,* 1996, vol. 14, 264-268
- **CHIU, R. W. K. et al.** *Lancet,* 2002, vol. 360, 998-1000
- **TANG, K. et al.** *Proc Natl Acad Sci USA,* 1999, vol. 96, 10016-10020
- **LO et al.** *Am J Hum Genet,* 1998, vol. 62, 768-775
- **DING AND CANTOR.** *Proc Natl Acad Sci USA,* 2003, vol. 100, 7449-7453
- **SUOMALAINEN et al.** *Mol. Biotechnol. Jun,* 2000, vol. 15 (2), 123-31
- **CHIU, R. W. K. et al.** *Clin Chem,* 2001, vol. 47, 1607-1613
- Molecular Cloning: A Laboratory Manual. Cold Spring Harbor Laboratory Press, 2001
- **SHALON et al.** *Genome Research,* 1996, vol. 6 (7), 639-45
- **BERNARD et al.** *Nucleic Acids Research,* 1996, vol. 24 (8), 1435-42
- **DORIS et al.** *J. Chromatogr. A,* 08 May 1998, vol. 806 (1), 47-60

- **HOLLAND et al.** *Proc Natl Acad Sci USA,* 1991, vol. 88, 7276-7280
- **HULTMAN et al.** *Nucl. Acid. Res.,* 1988, vol. 17, 4937-4946
- **SYVANEN et al.** *Genomics,* 1990, vol. 8, 684-692
- **WARTIOVAARA ; SYVANEN.** Quantitative analysis of human DNA sequences by PCR and solid-phase minisequencing. *Mol Biotechnol,* June 2000, vol. 15 (2), 123-131
- **LO, Y.M.D. et al.** *Am J Hum Genet,* 1998, vol. 62, 768-775
- **DING, C ; CANTOR, C. R.** *Proc Natl Acad Sci USA,* 2003, vol. 100, 7449-7453
- **TANG K. et al.** *Proc Natl Acad Sci USA,* 1999, vol. 96, 10016-10020
- **CHIU, R. W. K et al.** *Clin Chem,* 2002, vol. 48, 778-780
- **DING, C. ; CANTOR, C. R.** *Proc Natl Acad Sci USA,* 2003, vol. 100, 7449-7453
- **LAU, Y.L. et al.** *N Engl J Med,* 1997, vol. 336, 1298-1301
- **TANG, K. et al.** *Proc Natl Acad Sci U S A,* 1999, vol. 96, 10016-10020
- **ANKER, P. et al.** *Gastroenterology,* 1997, vol. 112, 1114-1120
- **LO, Y.M.D. et al.** *Lancet,* 1998, vol. 351, 1329-1330
- **NG, I. S. ; ONG, J. B. ; TAN, C. L. ; LAW, H. Y.** *Hum. Genet.,* 1994, vol. 94, 385-388
- **SANGUANSERMSRI, T. ; THANARATTANAKOM, P. ; STEGER, H. F. ; TONGSONG, T. ; CHANPRA-PAPH, P. ; WANPIRAK, C. ; SIRIWATANAPA, P. ; SIRICHOTIYAKUL, S. ; FLATZ, G.** *Hemoglobin,* 2001, vol. 25, 19-27
- **CHIU, R. W. K. ; POON, L. L. M. ; LAU, T. K. ; LE-UNG, T. N. ; WONG, E. M. C. ; LO, Y. M. D.** *Clin. Chem.,* 2001, vol. 47, 1607-1613
- **TANG, K. ; FU, D. J. ; JULIEN, D. ; BRAUN, A. ; CANTOR, C. R. ; KOSTER, H.** *Proc. Natl. Acad. Sci. USA,* 1999, vol. 96, 10016-10020
- **DING, C. ; CANTOR, C. R.** *Proc. Natl. Acad. Sci. USA,* 2003, vol. 100, 7449-7453
- **LAU, Y. L. ; CHAN, L. C. ; CHAN, Y. Y. ; HA, S. Y. ; YEUNG, C. Y. ; WAYE, J. S. ; CHUI, D. H.** *N. Engl. J. Med.,* 1997, vol. 336, 1298-1301
- **LIANG, R. ; LIANG, S. ; JIANG, N. H. ; WEN, X. J. ; ZHAO, J. B. ; NECHTMAN, J. F. ; STOMING, T. A. ; HUISMAN, T. H.** *Br. J. Haematol.,* 1994, vol. 86, 351-354
- **VAN DER SCHOOT, C. E. ; TAX, G. H. ; RIJNDERS, R. J. ; DE HAAS, M. ; CHRISTIAENS, G. C.** *Transfusion Med. Rev.,* 2003, vol. 17, 31-44
- **ZHONG, X. Y. ; HOLZGREVE, W. ; HAHN, S.** *Swiss Med. Wkly.,* 2001, vol. 131, 70-74
- **AVENT, N. D. ; FINNING, K. M. ; MARTIN, P. G. ; SOOTHILL, P. W.** *Vox Sanguinis,* 2000, vol. 78, 155-162

- **LO, Y. M. D.** *Lancet,* 2002, vol. 360, 998-1000
- **CHIU, R. W. K. ; LAU, T. K. ; CHEUNG,P. T. ; GONG, Z. Q. ; LEUNG, T. N. ; LO, Y. M. D.** *Clin. Chem.,* 2002, vol. 48, 778-780
- **BIANCHI, D. W.** *Clin. Chem.,* 2002, vol. 48, 689-690
- **DING, C. ; CANTOR, C. R.** *Proc. Natl. Acad. Sci. USA,* 2003, vol. 100, 3059-3064
- **LO, Y. M. D. ; LAU, T. K. ; ZHANG, J. ; LEUNG, T. N. ; CHANG, A. M. ; HJELM, N. M. ; ELMES, R. S. ; BIANCHI, D. W.** *Clin. Chem.,* 1999, vol. 45, 1747-1751
- **ZHONG, X. Y. ; BURK, M. R. ; TROEGER, C. ; JACKSON, L. R. ; HOLZGREVE, W. ; HAHN, S.** *Prenatal Diagn.,* 2000, vol. 20, 795-798
- **LO, Y. M. D. ; LEUNG, T. N. ; TEIN, M. S. ; SAR-GENT, I. L. ; ZHANG, J. ; LAU, T. K. ; HAINES, C. J. ; REDMAN, C. W.** *Clin. Chem.,* 1999, vol. 45, 184-188
- **ZHONG, X. Y. ; LAIVUORI, H. ; LIVINGSTON, J. C. ; YLIKORKALA, O. ; SIBAI, B. M. ; HOLZGREVE, W. ; HAHN, S.** *Am. J. Obstet. Gynecol.,* 2001, vol. 184, 414-419
- **LEUNG, T. N. ; ZHANG, J. ; LAU, T. K. ; HJELM, N. M. ; LO, Y. M. D.** *Lancet,* 1998, vol. 352, 1904-1905
- **BOTEZATU, I. ; SERDYUK, O. ; POTAPOVA, G. ; SHELEPOV, V. ; ALECHINA, R. ; MOLYAKA, Y. ; ANANEV, V. ; BAZIN, I. ; GARIN, A. ; NARIMANOV, M. et al.** *Clin. Chem.,* 2000, vol. 46, 1078-1084
- **ANGERT, R. M. ; LESHANE, E. S. ; YARNELL, R. W. ; JOHNSON, K. L. ; BIANCHI, D. W.** *Am. J. Obstet. Gynecol.,* 2004, vol. 190, 1087-1090
- **BIANCHI, D. W. ; ROMERO, R.** *J. Maternal Fetal Neonatal Med.,* 2003, vol. 14, 123-129
- **NELSON, J. L.** *Lancet,* 2001, vol. 358, 2011-2012
- **LO, Y. M. D. ; LO, E. S. ; WATSON, N. ; NOAKES, L. ; SARGENT, I. L. ; THILAGANATHAN, B. ; WAINSCOAT, J. S.** *Blood,* 1996, vol. 88, 4390-4395
- **LO, Y. M. D. ; PATEL, P. ; WAINSCOAT, J. S. ; SAMPIETRO, M. ; GILLMER, M. D. ; FLEMING, K. A.** *Lancet,* 1989, vol. 2, 1363-1365
- **LO, Y. M. D. ; PATEL, P. ; WAINSCOAT, J. S. ; FLEMING, K. A.** *Lancet,* 1990, vol. 335, 724
- **LO, Y. M. D. ; CHAN, L. Y. S. ; LO, K. W. ; LEUNG, S. F. ; ZHANG, J. ; CHAN, A. T. C. ; LEE, J. C. ; HJELM, N. M. ; JOHNSON, P. J. ; HUANG, D. P.** *Cancer Res.,* 1999, vol. 59, 1188-1191
- **ANKER, P. ; LEFORT, F. ; VASIOUKHIN, V. ; LYAUTEY, J. ; LEDERREY, C. ; CHEN, X. Q. ; STROUN, M. ; MULCAHY, H. E. ; FARTHING, M. J.** *Gastroenterology,* 1997, vol. 112, 1114-1120
- **SORENSON, G. D.** *Ann. N. Y. Acad. Sci.,* 2000, vol. 906, 13-16

- **LO, Y. M. D. ; TEIN, M. S. ; PANG, C. C. ; YEUNG, C. K. ; TONG, K. L. ; HJELM, N. M.** *Lancet,* 1998, vol. 351, 1329-1330